# EUROPÄISCHE PATENTANMELDUNG

(11) **EP 1 707 206 A1**
(43) Veröffentlichungstag der Anmeldung: **04.10.2006**
(21) Anmeldenummer: 06111982.2
(22) Anmeldetag: 30.03.2006
(51) Int. Cl.: A61K 31/495, A61P 25/28, A61P 31/18, A61P 33/06

(54) **Piperazinderivate zur inhibition von Beta-Sekretase, Cathepsin D, Plasmepsin ll und HIV-Protease und zur Behandlung von Malaria, Alzheimer und AIDS**

(30) Priorität: 31.03.2005 CH 5692005
(71) Anmelder: Speedel Experimenta AG, 4123 Allschwil (CH)
(72) Erfinder: Herold, Peter, 4055, Basel (CH); Mah, Robert, 4132, Muttenz (CH); Stutz, Stefan, 4053, Basel (CH); Stojanovic, Aleksandar, 4055, Basel (CH); Tschinke, Vincenzo, 4102, Binningen (CH); Marti, Christiane, 5400, Baden (CH); Schumacher, Christoph, 4126, Bettingen (CH)
(74) Vertreter: Maué, Paul Georg

(57) **Zusammenfassung**

Es wird die Verwendung substituierter Piperazine der allgemeinen Formeln (I) und (II) 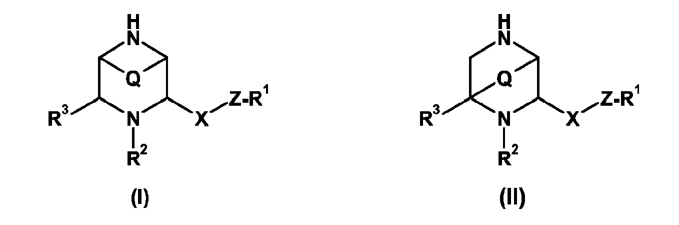 mit den Substituentenbedeutungen wie sie in der Beschreibung näher erläutert sind als Beta-Sekretase-, Cathepsin D-, Plasmepsin II- und/oder HIV-Protease-Hemmer beschrieben.

## Beschreibung

Die vorliegende Erfindung betrifft die Verwendung substituierter Piperazine als Beta-Sekretase-, Cathepsin D-, Plasmepsin 11- und/oder HIV-Protease-Hemmer.

Hinsichtlich insbesondere der Beta-Sekretase-, Cathepsin D-, Plasmepsin II- und/oder HIV-Protease-Hemmung besteht jedoch weiterhin ein Bedarf an hoch potenten Wirkstoffen. Hierbei steht die Verbesserung der pharmakokinetischen Eigenschaften im Vordergrund. Diese auf eine bessere Bioverfügbarkeit gerichteten Eigenschaften sind beispielsweise Absorption, metabolische Stabilität, Löslichkeit oder Lipophilie.

### Alzheimer Aspartyl Protease: Beta-Secretase

Die Alzheimer Krankheit ist eine progressive degenerative Krankheit des Gehirns. Die Symptome von Alzheimer sind progressiver Gedächtnisverlust, Sprachschwierigkeiten und schlussendlich Verlust der elementaren Nervenfunktion, die zum Tod führen kann. Als Biomarker sind im zentralen Nervensystem von Alzheimer Patienten Amyloid Plaques, intrazelluläre neurofibrilläre Vernetzungen "Tangles" und aktivierte Mikroglia zu finden. Das Auftreten dieser Biomarker trägt wahrscheinlich zum neuronalen Zelltod und Gedächtnisverlust in Alzheimer Patienten bei. Das beta-Amyloid ist ein definierendes Merkmal der Alzheimer Krankheit. Heute ist man der Meinung, dass es ein Mitverursacher ist und tragend das Fortschreiten der Krankheit beeinflusst. Das Auftreten von amyloiden Plaques und Angiopathien im Gehirn sind auch charakteristisch für Menschen mit Trisomy 21 (Down's Syndrom), Hereditary Cerebral Hemorrhage with Amloidosis of the Dutch-Type (HCHWA-D) und anderen neurodegenerativen Störungen.
Die beta-amyloiden Plaques bestehen vor allem aus dem Amyloid Beta-Peptid (A-Beta, manchmal auch als betaA4 bezeichnet). Das A-Beta Peptid entsteht proteolytisch vom Beta Amyloid Präkursor Protein (APP). Das Beta-APP wird durch drei geordnetnete enzymische Aktivitäten prozessiert. Der Grossteil des beta-APPs wird über die alpha-Sekretase in einem nicht-amyloidogenen Mechanismus verarbeitet. Ein kleiner Anteil des beta-APPs wird durch die beta-Sekretase Aktivität gespaltet um damit das membrangebundene carboxyterminale Fragment C99 zu bilden. Die gamma-Sekretase verarbeited das C99 Fragement zum amyloidogenen A-Beta Peptid, das aus 39-42 Aminosäuren besteht. Die Aspartyl-Protease Aktivität der Beta-Sekretase wurde bei Anwendung unterschiedlicher Nomenklatur auch als BACE (beta-site APP cleaving enzyme), Asp und Memapsin bezeichnet.

Die Bedeutung der proteolytischen Spaltung von Beta-APP durch die Beta-Sekretase, als entscheidender Schritt, in der Enstehung der Alzheimer Krankheit, ist durch die Beobachtung belegt, dass humane Mutationen in der Beta-Sekretase (Schwedische Mutanten) zu einer erhöhten A-Beta Peptid Erzeugung und somit zu einer frühen, vererbaren Form der Alzheimer Krankheit führen. Andererseits vermögen BACE1 defiziente Mäuse, die einen normalen Phenotypen zeigen, kein A-Beta Peptid zu bilden. Wenn diese Mäuse mit APP transgenen Mäusen, die durch einen Überschuss an APP gezeichnet sind, gekreuzt werden, kann bei den Nachkommen, im Vergleich zu BACE1 normalen Tieren, eine reduzierte Menge an A-Beta Peptid in Gehirnextrakten festgestellt werden. Diese Feststellungen unterstützen den Ansatz, dass die pharmakologische Hemmung der Beta-Sekretase und die einhergehende Verminderung der A-Beta Peptid Ablagerungen im Gehirn, eine therapeutische Strategy zur Behandlung der Alzheimer Krankheit und weiteren beta-amyloiden Syndromen darstellt.
Chemische Verbindungen, welche effektiv die Beta-Sekretase Aktivität hemmen, werden die durch die Beta-Sekretase erzeugte Spaltung von APP zum A-Beta Peptid inhibieren. Die pharmakologische Hemmung der A-Beta Peptid Erzeugung reduziert möglicherweise Beta-Amyloid Ansammlungen, respektive die Entstehung von Plaques. Beta-Sekretase hemmende chemische Verbindungen sind daher zur Behandlung oder zur Prevention von Krankheiten von Nutzen, welche durch Amyloid Beta Ansammlungen oder Ablagerungen charakterisiert sind. Die vorliegende Erfindung bezieht sich auch auf Methoden und Anwendung therapeutisch wirksamer pharmazeutischer Zusammensetzungen zur Behandlung von Patienten oder zur Prevention von Krankheiten. Die Methoden umfassen die Alzheimer Krankheit, die Prevention oder Verzögerung der Entwicklung von Alzheimer, die Behandlung von Patienten mit MCI (mild cognitive impairment), von Patienten, deren MCI zur Alzheimer Krankheit fortschreitet, die Behandlung des Down Syndromes, die Behandlung von HCHWAD, die Behandlung der zerebralen amyloiden Angiopathy, und die Behandlung degenerativer Hirnleistungsschwächen.

### Alzheimer Aspartyl Protease: Cathepsin D

Das humane Enzym Cathepsin D ist eine intrazelluläre Aspartyl Peptidase, welches hauptsächlich in Lysosomen gefunden wird. Cathepsin D reguliert verschiedene Haushaltsfunktionen des gesamten Metabolismus, unter anderem den Abbau von zellulären und phagozytierten Proteinen. Das Enzyme ist auch wahrscheinlich in einer Vielzahl von Krankheitszuständen involviert, so wie zum Beispiel in Krebs und der Alzheimer Krankheit. Klinische Studien haben gezeigt, dass Cathepsin D übermässig exprimiert in Brustkrebs-Zellen vorkommt und dies scheint, durch erhöhtes Zellwachstum, mit einem erhöhten Risiko zu Metastasen verbunden sein. Es wird auch vermutet, dass Cathepisin D in der Bildung des Beta-Amyloid Peptides in der Alzheimer Krnakheit involviert ist. Die Verfügbarkeit von selektiven und potenten Hemmern von Cathepsin D werden helfen, um weitere Funktionen des Cathepsin D Enzymes in Krankheitszuständen zu definieren und somit die Entwicklung von neuen therapeutischen Substanzen erleichtern.

### Malaria Aspartyl Protease: Plasmepsin I und II

Malaria wird als eine der ernsthaftesten Infektionskrankheiten der Welt betrachtet, welche rund 500 Millionen Personen betrifft. Die Krankheit wird durch die Anopheles-Mücke verbreitet, welche hauptsächlich in tropischen Regionen anzutreffen ist. Die Spezies Plasmodium Falziparum ist für mehr als 95% der mit Malaria verbundenen Morbiditäts- und Mortalitätsfälle verantwortlich. Das Plasmodium Falziparum ist zunehmend resistent gegen bestehende Behandlungsansätze wie Chloroquine, Mefloquine und Sulfadoxime/ Pyrimethamine. Daher besteht ein dringenden Bedarf für neue Behandlungsmöglichkeiten. Im erythrozytären Stadium des parasitären Lebenszyklus dringt der Parasit in die roten Blutzellen seines Gastgeber Orgnismus ein und verzehrt bis zu 80% des Hemoglobins als Nahrungsquelle für Wachstum und Entwicklung. Der Hemoglobinabbau findet in einer aziden Vakuole im Parasiten statt und viele der gegenwärtigen Malaria Medikamente scheinen diese wichtige Vakuolenfunktion zu beeinträchtigen. Die Vakuole enthält Aspartyl-, Cysteine-und Metallo-Proteasen, welche alle im Hemoglobinabbau involviert sein könnten. Mindestens 10 Gene zur Kodierung von Aspartyl-Proteasen wurden im Plasmodiumgenom identifiziert. Vier dieser Aspartyl-Proteasen wurden in den aziden Nahrungsmittelvakuole des Parasiten entdeckt, namentlich Plasmepsin I, II, IV und HAP, ein Histoaspartyl-Protease. Inhibitoren der Plasmepsin I und II Proteasen haben ihren Nutzen in Zell- und Tiermodellen für Malaria gezeigt, wodurch diese Enzyme als therapeutischer Ansatz zur Entdeckung von neuen Medikamenten validiert wurden. Ein nicht-selektiver Inhibitor der Aspartyl-Proteasen, Pepstatin, inhibiert das Wachstum von Plasmodium Falziparum in vitro. Ähnliche Resultate wurden mit Analogen von Pepstatin erziehlt. Diese Experimente zeigen, dass die Hemmung von Aspartyl-Proteasen den Lebenszyklus des Plasmodium Falziparum unterbrechen kann. Die dargestellten Erfindungen beziehen sich auf die Identifikation von niedermolekularen, nicht-peptidischen Inhibitoren der Plasmodium Falziparum Protease Plasmepsin II oder anderen Aspartyl-Proteasen zur Behandlung oder Verhinderung der Übertragung von Malaria.

### HIV Aspartyl Protease: HIV-1 Peptidase

Das im Jahre 1981 erstmals in einer kleinen Anzahl von Patienten festgestellte Acquired Immunodeficiency Syndrome (AIDS) ist heute eine bedeutende Epidemie mit weltweit mehr als 38 Millionen infizierten Menschen, von welchen rund 1 Million in den Vereinigten Staaten, 580'000 in West-Europa und mehr als 25 Millionen in Afrika leben (http://www.unaids.org). Seit dem Zeitpunkt als AIDS erstmals klinisch identifiziert wurde, hat zwar ein bedeutender wissenschaftlicher und therapeutischer Fortschritt stattgefunden, jedoch bleibt AIDS, vor allem in den Entwicklungsländern, eine nicht-kontrollierbare Krankheit.
Die Prognosen für AIDS Patienten, welche uneingeschränkten Zugang zu den heutigen Therapiemöglichkeiten haben, hat sich seit der Entdeckung von AIDS komplett verändert. Heute ist die durchschnittliche Lebenserwartung für behandelte HIV-positive Patienten über 8 Jahre. Bevor das Medikament AZT im Jahr 1987 eingeführt wurde, lag die Lebenserwartung von AIDS Patienten noch unter einem Jahr. Diese drastische Verbesserung der Lebenserwartung ist vor allem auf die Entwicklung von wirkungsvollen Therapien, auf die frühe Erkennung von Human Immunodeficiency Virus (HIV)-Trägern und auf das kontinuierliche Bestreben deer Verminderung der Virenresistenz durch die Einfuhrung neuer Medikamente und Kombinationstherapien, zurückzuführen.
Die von der FDA bewilligten Therapien hemmen drei Mechanismen des HIV-Lebenszyklus, nämlich die reverse Transkription, die proteolytische Reifung und die Zellfusion. Dreifachtherapien, gewöhnlich als Highly Active Antiretroviral Therapie (HAART) bekannt, stellen heute den Behandlungsstandard dar. Diese Behandlungsmethode schreibt die Anwendung eines Protease Inhibitors oder eines Non-Nucleoside Reverse Transkriptase Inhibitors in Kombination mit zwei Nucleoside Reverse Transkriptase Inhibitoren vor.
Die Translation der HIV Ribonukleinsäure führt zur Erzeugung von zwei Polyprotein Vorstufen, das Gag und das Gag-Pol. Das 55-kDa grosse Gag Polyprotein beinhaltet die viralen Strukturproteine und das 160-kDa grosse Gag-Pol Polyprotein enthält die funkionellen viralen Enzyme Reverse Transcriptase, Protease und Integrase. Die exprimierten Gag und Gag-Pol Polyproteine werden zur Plasmamembran transportiert, wo üblicherweise eine Ansammlung von Typ-C Retroviren und Lentiviren entsteht. Während der Ansammlung von Partikeln zerteilt die virale Protease die Gag und Gag-Pol Vorstufen in die, für die Virenvermehrung essentiellen, strukturellen und funktionellen Proteine. Die Protease Aktivität im Zytoplasma von infizierten Zellen erlaubt es das Viruspartikel zu bilden und von der Zelle durch Knospung freizusetzen und zu verbreiten.

Die aktive HIV-1 Protease bildet ein Dimer aus zwei identischen, 11 kDa grossen Untereinheiten, welche je ein katalytisches Aspartyl beitragen. Im Gegensatz dazu sind nichtvirale, dh. von humanen Zellen gebildete Mitglieder der Aspartyl-Protease Familie monomere Enzyme, die zwei Asp-Thr-Gly Sequenzbereiche enthalten. Die einzigartige dimere Struktur der retroviralen Protease wird hauptsächlich durch eine antiparallele Beta-Faltblattstruktur stabilisiert, welche durch Verzahnung der Untereinheiten an deren amino- und carboxyterminalen Enden geformt wird.

Die Aktivierung der HIV-1 Protease durch die Dimerisierung oder durch die autokatalytischen Freigabe des Gag-Pol Polyproteins ist ein kritischer Schritt im viralen Lebenszyklus. So führt die Hemmung der viralen Proteaseaktivität zur Hinderung der Gag Polyproteinverarbeitung, Virusbildung und zum Verlust der viralen Infektiosität. Somit sind virale Proteasehemmer eine wichtige therapeutische Strategie gegen HIV, die zu mehreren Medikamenten geführt hat. Das Design dieser Medikamente wurde durch den Zugang zu Kristallstrukturen, die entweder das nackte Enzym oder den Enzym-Inhibitorkomplex darstellen, stark erleichtert. Zudem sind heutzutage ausführliche biochemische Daten vorhanden, welche die katalytischen Mechanismen und die molekulare Basis der enzymatischen Substratbindung ausführlich schildern.

Ein Gegenstand der Erfindung ist daher die Verwendung substituierter Piperazinderivate der allgemeinen Formeln (I) und (II) 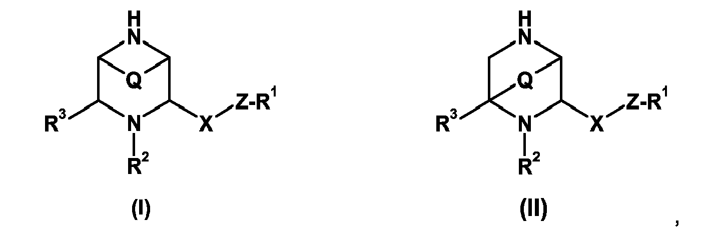 worin
R¹ Aryl oder Heterocyclyl;
R² Phenyl, Naphthyl, Acenaphthyl, Cyclohexyl, Pyridyl, Pyrimidinyl, Pyrazinyl, Oxo-pyridinyl, Diazinyl, Triazolyl, Thienyl, Oxazolyl, Oxadiazolyl, Thiazolyl, Pyrrolyl, Furyl, Tetrazolyl oder Imidazolyl, welche Reste durch 1-3 Halogen, Hydroxy, Cyano, Trifluormethyl, C₁₋₆-Alkyl, Halo-C₁₋₆-alkyl, Hydroxy-C₁₋₆-alkyl, C₁₋₆-Alkoxy-C₁₋₆-alkyl, Cyano-C₁₋₆-alkyl, Carboxy-C₁₋₆₋alkyl, C₁₋₆-Alkanoyloxy-C₁₋₆-alkyl, C₁₋₆-Alkoxycarbonyloxy-C₁₋₆-alkyl, C₁₋₆-Alkoxycarbonyl, oder C₁₋₆-Alkoxygruppen, oder eine C₁₋₆-Alkylendioxygruppe, und/oder durch einen Rest L1-T1-L2-T2-L3-T3-L4-T4-L5-U substituiert sein können, darstellen;
L1, L2, L3, L4 und L5 unabhängig voneinander eine Bindung, C₁₋₈-Alkylen, C₂₋₈-Alkenylen oder C₂₋₈-Alkinylen darstellen, oder abwesend sind;
T1, T2, T3 und T4 unabhängig voneinander
(a) eine Bindung darstellen, oder abwesend sind, oder eine der Gruppen
(b) -CH(OH)-,
(c) -CH(OR⁶)-,
(d) -CH(NR⁵R⁶)-,
(e) -CO-,
(f) -CR⁷R⁸-,
(g) -O- oder -NR⁶-,
(h) -S(O)₀₋₂-,
(i) -SO₂NR⁶-,
(j) -NR⁶SO₂-,
(k) -CONR⁶-,
(I) -NR⁶CO-,
(m) -O-CO-,
(n) -CO-O-,
(o) -O-CO-O-,
(p) -O-CO-NR⁶-,
(q) -N(R⁶)-CO-N(R⁶)-,
(r) -N(R⁶)-CO-O-,
(s) Pyrrolidinylen, Piperidinylen oder Piperazinylen, und
(t) -C(R¹¹)(R¹²)-
darstellen,
wobei die von (b)-(t) ausgehenden Bindungen zu einem gesättigten oder aromatischen C-Atom der benachbarten Gruppe führen, falls die Bindung von einem Heteroatom ausgeht, und wobei nicht mehr als zwei Gruppen (b)-(f), drei Gruppen (g)-(h) und eine Gruppe (i)-(t) anwesend sind;
R³ Wasserstoff, C₁₋₆-Alkyl, C₂₋₆-Alkenyl, C₁₋₆-Alkoxy, Hydroxy-C₁₋₆-alkyl, C₁₋₆-Alkoxy-C₁₋₆-alkyl, Benzyl, Oxo, oder eine Gruppe R⁴-Z1-X1- darstellen, wobei R⁴
(a) H-,
(b) C₁₋₆-Alkyl-,
(c) C₂₋₆-Alkenyl-,
(d) Hydroxy-C₁₋₆-alkyl-,
(e) Polyhydroxy-C₁₋₆-alkyl-,
(f) C₁₋₆-Alkyl-O-C₁₋₆-alkyl-,
(g) Aryl-,
(h) Heterocyclyl-,
(i) Arylalkyl-,
(j) Heterocyclylalkyl-,
(k) Aryloxyalkyl-,
(l) Heterocyclyloxyalkyl-,
(m) (R⁵, R⁶)N-(CH₂)₁₋₃-,
(n) (R⁵,R⁶)N-,
(o) C₁₋₆-Alkyl-S(O)₀₋₂-,
(p) Aryl-S(O)₀₋₂-,
(q) Heterocyclyl-S(O)₀₋₂-,
(r) HO-SO₃- bzw. deren Salze,
(s) H₂N-C(NH)-NH-, und
(t) NC- darstellt,
und die von (n)-(t) ausgehenden Bindungen zu einem C-Atom der benachbarten Gruppe führen und dieses C-Atom gesättigt ist, falls die Bindung von einem Heteroatom ausgeht;
Z1
(a) eine Bindung darstellt, abwesend ist, oder eine der Gruppen
(b) C₁₋₆-Alkylen-,
(c) C₂₋₆-Alkenylen-,
(d) -O-, -N(R¹¹)-, -S(O)₀-₂-,
(e) -CO-,
(f) -O-CO-,
(g) -O-CO-O-,
(h) -0-CO-N(R¹¹)-,
(i) -N(R¹¹)-CO-O-,
(j) -CO-N(R¹¹)-,
(k) -N(R¹¹)-CO-,
(I) -N(R¹¹)-CO-N(R¹¹)-,
(m) -CH(OR⁹)- darstellt,
und die von (d) und (f)-(m) ausgehenden Bindungen zu einem C-Atom der benachbarten Gruppe führen und dieses C-Atom gesättigt ist, falls die Bindung von einem Heteroatom ausgeht;
X1 eine Bindung darstellt, abwesend ist, oder -(CH₂)₁₋₃- darstellt;
R⁵ und R⁶ Wasserstoff, C₁₋₆-Alkyl, C₂₋₆-Alkenyl, Aryl-C₁₋₆-alkyl oder Acyl bedeuten, oder gemeinsam mit dem N-Atom, an das sie gebunden sind, einen 5- oder 6-gliedrigen heterocyclischen Ring bedeuten, der ein zusätzliches N-, O- oder S-Atom oder eine -SO- oder -SO₂- Gruppe enthalten kann, wobei das zusätzliche N-Atom gegebenenfalls durch C₁₋₆-Alkyl-Reste substituiert sein kann;
R⁷ und R⁸ gemeinsam mit dem C-Atom, an das sie gebunden sind, einen 3-7-gliedrigen Ring darstellen, der ein oder zwei -O- oder -S- Atome oder -SO- oder -SO₂- Gruppen enthalten kann;
R⁹ Wasserstoff, C₁₋₆-Alkyl, C₃₋₈-Cycloalkyl, C₁₋₆-Alkoxy-C₁₋₆-alkyl, Acyl oder Arylalkyl;
R¹⁰ Carboxyalkyl, Alkoxycarbonylalkyl, Alkyl oder Wasserstoff;
R¹¹ Wasserstoff oder C₁₋₆-Alkyl ist;
R¹² Wasserstoff oder C₁₋₆-Alkyl ist;
U Wasserstoff, C₁₋₆-alkyl, Cycloalkyl, Cyano, gegebenenfalls substituiertes Cycloalkyl, Aryl, oder Heterocyclyl darstellt;
Q Ethylen darstellt oder abwesend ist (Formel I) oder Ethylen oder Methylen darstellt (Formel II);
X eine Bindung oder eine Gruppe >CH-R¹¹, >CR⁹R¹¹, >CHOR⁹, >CO oder >C=NOR¹⁰ darstellt;
Z abwesend ist oder C₁₋₆-Alkylen, C₂₋₆-Alkenylen, Hydroxy-C₁₋₆-alkyliden, -CH-R¹¹-CO-NR⁹-, -O-, -S-, -NR⁹, -O-Alk-, -S-Alk-, -NR⁹-Alk-, -Alk-O-, -Alk-S- oder -Alk-NR⁹- ist, wobei Alk C₁₋₆-Alkylen bezeichnet; und wobei
(a) falls Z -O- oder-S- darstellt, X -CR⁹R¹¹- ist und entweder R² einen Substituenten L1-T1-L2-T2-L3-T3-L4-T4-L5-U enthält oder R³ ein wie oben definierter, von Wasserstoff verschiedener Substituent ist;
(b) falls Z -O-Alk-, -S-Alk- oder -NR⁹-Alk- darstellt, X -CR⁹R¹¹- ist; und
(c) falls X eine Bindung darstellt, Z C₁₋₆-Alkenylen, -Alk-O-, Alk-S- oder -Alk-NR⁹-darstellt, und pharmazeutisch anwendbare Salze davon.

Beispiele für C₁₋₆-Alkyl- und Alkoxyreste sind Methyl, Ethyl, n-Propyl, Isopropyl, n-Butyl, Isobutyl, sec.-Butyl, tert.-Butyl, Pentyl, Hexyl bzw. Methoxy, Ethoxy, Propoxy, Isopropoxy, Butoxy, Isobutoxy, sec.-Butoxy and tert.-Butoxy. C₁₋₆-Alkylendioxyreste sind vorzugsweise Methylendioxy, Aethylendioxy und Propylendioxy. Beispiele von C₁₋₆-Alkanoylresten sind Acetyl, Propionyl und Butyryl. Cycloalkyl bedeutet einen gesättigten, cyclischen Kohlenwasserstoffreste mit 3-6 Kohlenstoffatomen, d.h. Cyclopropyl, Cyclobutyl, Cyclopentyl und Cyclohexyl. C₁₋₈-Alkylenreste sind z.B. Methylen, Ethylen, Propylen, 2-Methylpropylen, Tetra-, Penta- und Hexamethylen; C₂₋₈-Alkenylenreste sind z.B. Vinylen und Propenylen; C₂₋₈-Alkinylenreste ist z.B. Ethinylen; Acylreste sind Alkanoylreste, vorzugsweise C₁₋₆-Alkanoylreste, oder Aroylreste, wie Benzoyl. Aryl bezeichnet ein- oder mehrkernige aromatische Reste, die ein- oder mehrfach substituiert sein können, wie beispielsweise Phenyl, substituiertes Phenyl, Naphthyl, substituiertes Naphthyl, Tetrahydronaphthyl oder substituiertes Tetrahydronaphthyl. Beispiele für Substituenten an solchen Arylresten sind C₁₋₆-Alkyl, Trifluormethyl, Nitro, Amino, C₁₋₆-Alkenyl, C₁₋₆-Alkoxy, C₁₋₆-Alkylcarbonyloxy, Hydroxy, Halogen, Cyano, Carbamoyl, Carboxy und C₁₋₆-Alkylendioxy, sowie gegebenenfalls durch Halogen, C₁₋₆-Alkyl, C₁₋₆-Alkoxy oder Dihydroxy-C₁-₆-alkylaminocarbonyl substituiertes Phenyl, Phenoxy, Phenylthio, Phenyl-C₁₋₆-alkyl oder Phenyl-C₁₋₆-alkoxy. Weitere Beispiele von Substituenten an Aryl- oder Heterocyclylresten sind C₁₋₆-Alkoxycarbonylphenyl, Hydroxy-C₁₋₆-alkylphenyl, Benzyloxy, Pyridylcarbonylamino-C₁₋₆-alkyl, C₁₋₆-Alkenyloxy, C₁₋₆-Alkoxy-C₁₋₆-alkoxy, C₁₋₆-Alkoxy-C₁₋₆-alkoxy-C₁₋₆-alkyl, Methoxybenyloxy, Hydroxybenzyloxy, Phenaethyloxy, Methylendioxybenzyloxy, Dioxolanyl-C₁₋₆-alkoxy, Cyclopropyl-C₁₋₆-alkyl, Cyclopropyl-C₁₋₆-alkoxy, Hydroxy-C₁₋₆-alkoxy, Carbamoyloxy-C₁₋₆-alkoxy, Pyridyl-carbamoyloxy-C₁₋₆-alkoxy, Benzoyloxy-C₁₋₆-alkoxy, C₁₋₆-Alkoxy-carbonyl, C₁₋₆-Alkylcarbonylamino, C₁₋₆-Alkylcarbonylamino-C₁₋₆-alkyl, C₁₋₆-Alkylcarbonylamino-C₁₋₆-alkoxy, (N-C₁₋₆-Alkyl)-C₁₋₆-alkylcarbonylamino-C₁₋₆-alkyl, (N-C₁₋₆-Alkyl)-C₁₋₆-alkylcarbonylamino-C₁₋₆-alkoxy, C₃₋₆-Cycloalkylcarbonylamino-C₁₋₆-alkyl, C₃₋₆-Cycloalkylcarbonylamino-C₁₋₆-alkoxy, C₁₋₆-Alkoxy-C₁₋₆-alkyl, Hydroxy-C₁₋₆-alkyl, Hydroxy-C₁₋₆-alkoxy-C₁₋₆-alkyl, Hydroxy-C₁₋₆-alkoxy-C₁₋₆-alkoxy, C₁₋₆-Alkoxycarbonylamino-C₁₋₆-alkyl, C₁₋₆-Alkoxycarbonylamino-C₁₋₆-alkoxy, C₁₋₆-Alkylaminocarbonylamino-C₁₋₆-alkyl, C₁₋₆-Alkylaminocarbonylamino-C₁₋₆-alkoxy, C₁₋₆-Alkylaminocarbonyl-C₁₋₆-alkyl, C₁₋₆-Alkylaminocarbonyl-C₁₋₆-alkoxy, C₁₋₆-Alkylaminocarbonyl-C₁₋₆-alkoxy-C₁₋₆-alkyl, Di-C₁₋₆-alkylaminocarbonyl-C₁₋₆-alkyl, Di-C₁₋₆-alkylaminocarbonyl-C₁₋₆-alkoxy, C₁₋₆-Alkylcarbonyloxy-C₁₋₆-alkyl, C₁₋₆-Alkylcarbonyloxy-C₁₋₆-alkoxy, Cyano-C₁₋₆-alkyl, Cyano-C₁₋₆-alkoxy, 2-Oxo-oxazolidinyl-C₁₋₆-alkyl, 2-Oxo-oxazolidinyl-C₁₋₆-alkoxy, C₁₋₆-Alkoxycarbonyl-C₁₋₆-alkyl, C₁₋₆-Alkoxycarbonyl-C₁₋₆-alkoxy, C₁₋₆-Alkylsulfonylamino-C₁₋₆-alkyl, C₁₋₆-Alkylsulfonylamino-C₁₋₆-alkoxy, (N-C₁₋₆-Alkyl)-C₁₋₆-alkylsulfonylamino-C₁₋₆-alkyl, (N-C₁₋₆-Alkyl)-C₁₋₆-alkylsulfonylamino-C₁₋₆₋alkoxy, C₁₋₆-Alkylamino-C₁₋₆-alkyl, C₁₋₆-Alkylamino-C₁₋₆-alkoxy, Di-C₁₋₆-alkylamino-C₁₋₆-alkyl, Di-C₁₋₆-alkylamino-C₁₋₆-alkoxy, C₁₋₆-Alkylsulfonyl-C₁₋₆-alkyl, C₁₋₆-Alkylsulfonyl-C₁₋₆-alkoxy, Carboxy-C₁₋₆-alkyl, Carboxy-C₁₋₆-alkoxy, Carboxy-C₁₋₆-alkoxy-C₁₋₆-alkyl, C₁₋₆-Alkoxy-C₁₋₆₋alkyl-carbonyl, Acyl-C₁₋₆-alkoxy-C₁₋₆-alkyl, (N-C₁₋₆-Alkyl)-C₁₋₆-alkoxy-carbonylamino, (N-Hydroxy)-C₁₋₆-alkylaminocarbonyl-C₁₋₆-alkyl, (N-Hydroxy)-C₁₋₆-alkylaminocarbonyl-C₁₋₆₋alkoxy, (N-Hydroxy)-aminocarbonyl-C₁₋₆-alkyl, (N-Hydroxy)-aminocarbonyl-C₁₋₆-alkoxy, C₁₋₆-Alkoxy-aminocarbonyl-C₁₋₆-alkyl, 6-Alkoxy-aminocarbonyl-C₁₋₆-alkoxy, (N-C₁₋₆-alkoxy)-C₁₋₆-alkylaminocarbonyl-C₁₋₆-alkyl, (N-C₁₋₆-alkoxy)-C₁₋₆-alkylaminocarbonyl-C₁₋₆-alkoxy, (N-Acyl)-C₁₋₆-alkoxy-C₁₋₆-alkylamino, C₁₋₆-Alkoxy-C₁₋₆-alkyl-carbamoyl, (N-C₁₋₆-Alkyl)-C₁₋₆-alkoxy-C₁₋₆-alkyl-carbamoyl, C₁₋₆-Alkoxy-C₁₋₆-alkyl-carbonyl, C₁₋₆-Alkoxy-C₁₋₆-alkyl-carbonylamino, (N-C₁₋₆-alkyl)-C₁₋₆-Alkoxy-C₁₋₆-alkyl-carbonylamino, 1-C₁₋₆-Alkoxy-C₁₋₆-alkyl-imidazol-2-yl, 1-C₁₋₆-Alkoxy-C₁₋₆-alkyl-tetrazol-5-yl, 5-C₁₋₆-Alkoxy-C₁₋₆-alkyl-tetrazol-1-yl, 2-C₁₋₆-Alkoxy-C₁₋₆-alkyl-4-oxo-imidazol-1-yl, Carbamoyl-C₁₋₆-alkyl, Carbamoyl-C₁₋₆-alkoxy, C₁₋₆-Alkyl-carbamoyl, Di-C₁₋₆-alkyl-carbamoyl, C₁₋₆-Alkylsulfonyl, C₁₋₆-Alkyl-amidinyl, Acetamidinyl-C₁₋₆-alkyl, O-Methyloximyl-C₁₋₆-alkyl, O,N-Dimethyl-hydroxylamino-C₁₋₆-alkyl, sowie gegebenenfalls durch Halogen, C₁₋₆-Alkyl, C₁₋₆-Alkoxy oder Dihydroxy-C₁₋₆-alkylaminocarbonyl substituiertes Pyridyl, Pyridyloxy, Pyridylthio, Pyridylamino, Pyridyl-C₁₋₆-alkyl, Pyridyl-C₁₋₆-alkoxy, Pyrimidinyl, Pyrimidinyloxy, Pyrimidinylthio, Pyrimidinylamino, Pyrimidinyl-C₁₋₆-alkyl, Pyrimidinyl-C₁₋₆-alkoxy, Thienyl, Thienyl-C₁₋₆-alkyl, Thienyl-C₁₋₆-alkoxy, Furyl, Furyl-C₁₋₆-alkyl, Furyl-C₁₋₆-alkoxy.
Der Ausdruck Heterocyclyl bezeichnet mono- oder bicyclische, gesättigte und ungesättigte heterocyclische Reste mit 1 bis 4 Stickstoff- und/oder 1 oder 2 Schwefel- oder Sauerstoffatomen, die ein- oder mehrfach, insbesondere durch (im Falle ungesättigter Heterocyclyl-reste) Alkyl, Hydroxy, Alkoxy, Nitro oder Halogen oder durch Substituenten, wie oben für Arylreste definiert, substituiert sein können oder (im Falle gesättigter Heterocyclreste) durch Alkyl oder Alkoxy substituiert sein können.
Beispiele für Heterocyclyl-Reste sind Pyridyl, Thienyl, Pyrazinyl, Triazolyl, Imidazolyl, Benzthiazolyl, Furyl, Pyrimidinyl, Morpholinyl, Chinazolinyl, Chinolyl, Chinoxalinyl, Isochinolyl, Benzo[b]thienyl, Isobenzofuranyl, Benzimidazolyl, 2-Oxo-benzimidazolyl, Oxazolyl, Thiazolyl, Indolyl, Pyrrolyl, 2-Oxo-dihydro-benzo-[d][1,3]oxazinyl, 4-Oxo-dihydroimidazolyl, 5-Oxo-4H-[1,2,4]triazinyl, 3-Oxo-4H-benzo[1,4]thiazinyl, Tetrahydro-quinoxalinyl, 1,1,3-Trioxo-dihydro-2H-1lambda*6*-benzo[1,4]thiazinyl, 1-Oxo-pyridyl, Dihydro-3H-benzo[1,4]oxazinyl, 2-Oxo-tetrahydro-benzo[e][1,4]diazepinyl, 2-Oxo-dihydro-benzo[e][1,4]diazepinyl, 1H-Pyrrolizinyl, Phthalazinyl, 1-Oxo-3H-isobenzofuranyl, 4-Oxo-3H-thieno[2,3-d]pyrimidinyl, 3-Oxo-4H-benzo[1,4]oxazinyl, [1,5]Naphthyridyl, Dihydro-2H-benzo[1,4]thiazinyl, 1, 1 -Dioxo-dihydro-2H-benzo[1,4]thiazinyl, 2-Oxo-1 H-pyrido[2,3-b][1,4]oxazinyl, Dihydro-1 H-pyrido[2,3-b][1,4]oxazinyl, 1 H-Pyrrolo[2,3-b]pyridyl, Benzo[1,3]dioxolyl, Benzooxazolyl, 2-Oxo-benzooxazolyl, 2-Oxo-1,3-dihydroindolyl, 2,3-Dihydroindolyl, Indazolyl oder Benzofuranyl.
Beispiele substituierter Heterocyclreste sind Nitrobenzthiazolyl, Phenyl-tetrazolyl, Phenyl-oxadiazolyl, Phenyl-piperidinyl, Phenyl-piperazinyl, Phenyl-pyrrolidinyl, Thienyl-oxadiazolyl, Furanyl-oxadiazolyl, Benzyl-oxadiazolyl oder Phenyl-oxazolyl. Beispiele gesättigter Heterocyclyl-Reste sind Dioxolanyl, Dioxanyl, Dithiolanyl, Dithianyl, Pyrrolidinyl, Piperidinyl, Piperazinyl, 4-Methylpiperazinyl, Morpholinyl, Thiomorpholinyl, 2-Hydroxymethylpyrrolidinyl, 3-Hydroxypyrrolidinyl, 3,4-Dihydroxypyrrolidinyl, 4-Hydroxypiperidinyl, 4-Oxopiperidinyl, 3,5-Dimethylmorpholinyl, 4,4-Dioxothiomorpholinyl, 4-Oxothiomorpholinyl, 2,6-Dimethylmorpholinyl, Tetrahydropyranyl, 2-Oxo-imidazolidinyl, 2-Oxo-oxazolidinyl, 2-Oxo-piperidinyl, 2-Oxo-pyrrolidinyl, 2-Oxo-[1,3]oxazinyl, 2-Oxoazepanyl, 2-Oxo-tetrahydro-pyrimidinyl und dergleichen.
Im Falle von R¹, R⁴ und R⁹ können die Aryl, Aroyl- und Heterocyclylreste zusätzlich noch durch Heterocyclylalkyl, Heterocyclylalkoxy, Heterocyclylalkoxyalkyl oder Heterocyclyl wie beispielsweise Piperidinoalkyl, Piperidinoalkoxy, Piperidinoalkoxyalkyl, Morpholinoalkyl, Morpholinoalkoxy, Morpholinoalkoxyalkyl, Piperazinoalkyl, Piperazinoalkoxy, Piperazinoalkoxyalkyl, [1,2,4]-Triazol-1-yl-alkyl, [1,2,4]-Triazol-1-yl-alkoxy, [1,2,4]-Triazol-4-yl-alkyl, [1,2,4]-Triazol-4-yl-alkoxy, [1,2,4]-Oxadiazol-5-yl-alkyl, [1,2,4]-Oxadiazol-5-yl-alkoxy, 3-Methyl-[1,2,4]-oxadiazol-5-yl-alkyl, 3-Methyl-[1,2,4]-oxadiazol-5-yl-alkoxy, 5-Methyl-[1,2,4]-oxadiazol-3-yl-alkyl, 5-Methyl-[1,2,4]-oxadiazol-3-yl-alkoxy, Tetrazol-1-yl-alkyl, Tetrazol-1-yl-alkoxy, Tetrazol-2-yl-alkyl, Tetrazol-2-yl-alkoxy, Tetrazol-5-yl-alkyl, Tetrazol-5-yl-alkoxy, 5-Methyl-tetrazol-1-yl-alkyl, 5-Methyl-tetrazol-1-yl-alkoxy, Thiazol-4-yl-alkyl, Thiazol-4-yl-alkoxy, Oxazol-4-yl-alkyl, Oxazol-4-yl-alkoxy, 2-Oxo-pyrrolidinyl-alkyl, 2-Oxo-pyrrolidinyl-alkoxy, Imidazolyl-alkyl, Imidazolyl-alkoxy, 2-Methyl-imidazolyl-alkyl, 2-Methyl-imidazolyl-alkoxy oder N-Methylpiperazinoalkyl, N-Methylpiperazinoalkoxy, N-Methylpiperazinoalkoxyalkyl, sowie Alkylaminoalkyl, Alkylaminoalkoxy, Alkylaminoalkoxyalkyl, Mono- und Polyhydroxy-alkyl, -alkoxy, -alkoxyalkyl und -alkoxyalkoxy, Carbamoylalkyloxy, C₁₋₆-Alkoxy, Amino-C₁₋₆-alkoxy, Hydroxy-C₁₋₆-alkoxy, Dioxolanyl, Dioxanyl, Dithiolanyl, Dithianyl, Pyrrolidinyl, Piperidinyl, Piperazinyl, Pyrrolyl, 4-Methylpiperazinyl, Morpholinyl, Thiomorpholinyl,
2-Hydroxymethylpyrrolidinyl, 3-Hydroxypyrrolidinyl, 3,4-Dihydroxypyrrolidinyl, 3-Acetamidomethylpyrrolidinyl, 3-C₁₋₆-Alkoxy-C₁₋₆-alkyl-pyrrolidinyl, 4-Hydroxypiperidinyl, 4-Oxopiperidinyl, 3,5-Dimethylmorpholinyl, 4,4-Dioxothiomorpholinyl, 4-Oxothiomorpholinyl, 2,6-Dimethylmorpholinyl, 2-Oxo-imidazolidinyl, 2-Oxo-oxazolidinyl, 2-Oxo-pyrrolidinyl, 2-Oxo-[1,3]oxazinyl, 2-Oxo-tetrahydro-pyrimidinyl und dergleichen oder durch den Rest-O-CH₂CH(OH)CH₂NRₓ substituiert sein, wobei NRₓ einen mono- oder di-C₁₋₆-Alkyl-amino-, Piperidino-, Morpholino-, Piperazino- oder N-Methylpiperazinorest darstellt.
Beispiele für durch NR⁵R⁶ dargestellte 5- und 6-gliedrige heterocyclische Ringe sind Pyrrolidinyl, Piperidinyl, Piperazinyl, Morpholinyl, Thiomorpholinyl,
2-Hydroxymethylpyrrolidinyl, 3-Hydroxypyrrolidinyl, 3,4-Dihydroxypyrrolidinyl, 4-Hydroxypiperidinyl, 4-Oxopiperidinyl, 3,5-Dimethylmorpholinyl, 4,4-Dioxothiomorpholinyl, 4-Oxothiomorpholinyl, 2,6-Dimethylmorpholinyl, 2-Oxo-imidazolidinyl, 2-Oxo-oxazolidinyl, 2-Oxo-pyrrolidinyl, 2-Oxo-[1,3]oxazinyl, 2-Oxo-tetrahydro-pyrimidinyl und dergleichen. Beispiele für durch CR⁷R⁸ dargestellte 3-7-gliedrige Ringe sind Cyclopentyl, Cyclohexyl, Cycloheptyl, 1,3-Dioxolanyl, 1,3-Dioxanyl, 1,3-Dithiolanyl und 1,3-Dithianyl.
Der Ausdruck Polyhydroxyalkyl bezeichnet C₁-C₇-Alkylreste, die mit 2-6 Hydroxy-Gruppen substituiert sein können, wie z.B. Glyceryl, Arabityl, Sorbityl usw.

Die Verbindungen der Formel (I) oder Formel (II) besitzen mindestens ein asymmetrisches Kohlenstoffatom und können deshalb in Form von optisch reinen Enantiomeren, Enantiomerengemischen oder als Racemate vorliegen. Verbindungen mit einem zweiten asymmetrischen Kohlenstoffatom können in Form von optisch reinen Diastereomeren, Diastereomerengemischen, diastereomeren Racematen, Gemischen von diastereomeren Racematen oder als Mesoverbindungen vorliegen. Die Erfindung umfasst alle diese Formen. Enantiomerengemische, Racemate, Diastereomerengemische, diastereomere Racemate oder Gemische von diastereomeren Racematen können nach üblichen Methoden aufgetrennt werden, z.B. durch Racematspaltung, Säulenchromatographie, Dünnschichtchromatographie, HPLC und dergleichen.

Der Ausdruck "pharmazeutisch verwendbare Salze" umfasst Salze mit anorganischen oder organischen Säuren, wie Salzsäure, Bromwasserstoffsäure, Salpetersäure, Schwefelsäure, Phosphorsäure, Zitronensäure, Ameisensäure, Maleinsäure, Essigsäure, Bernsteinsäure, Weinsäure, Methansulfonsäure, p-Toluolsulfonsäure und dergleichen.

Die im folgenden genannten Verbindungsgruppen sind nicht als geschlossen zu betrachten, sondern es können in sinnvoller Weise, z.B. zur Ersetzung allgemeiner durch speziellere Definitionen, Teile dieser Verbindungsgruppen untereinander oder durch die oben gegebenen Definitionen ausgetauscht oder weggelassen werden.

Bevorzugte erfindungsgemässe Verbindungen sind solche der allgemeinen Formeln (IA) oder (IIA) 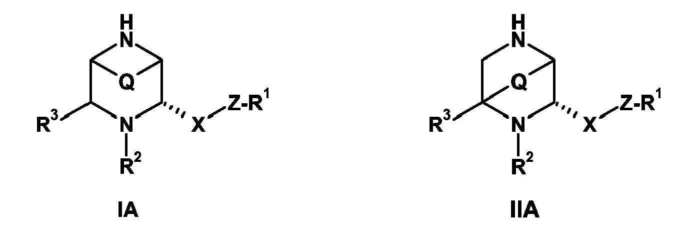 worin R¹, R², R³, Q, X und Z die für die Verbindungen der Formeln (I) bzw. (II) oben angegebene Bedeutung haben.

Eine weitere, bevorzugte Gruppe von Verbindungen der Formel (I) oder (II), bzw. besonders bevorzugt der Formel (IA) oder (IIA), sind Verbindungen worin
R¹ Aryl oder Heterocyclyl,
R² Phenyl, Cyclohexyl, Tetrazolyl, durch Halogen, Hydroxy, Cyano, Trifluormethyl, C₁₋₆-Alkyl, Halo-C₁₋₆-alkyl, Hydroxy-C₁₋₆-alkyl, C₁₋₆-Alkoxy-C₁₋₆-alkyl, Cyano-C₁₋₆-alkyl, Carboxy-C₁₋₆₋alkyl, C₁₋₆-Alkanoyloxy-C₁₋₆-alkyl, C₁₋₆-Alkoxycarbonyloxy-C₁₋₆-alkyl, C₁₋₆-Alkoxycarbonyl, C₁₋₆-Alkoxy, C₁₋₆-Alkylendioxy, oder durch einen Rest L1-T1-L2-T2-L3-T3-L4-T4-L5-U substituiertes Phenyl, Cyclohexyl oder Tetrazolyl; oder Naphthyl oder Acenaphthyl;
L1, L2, L3, L4 und L5 unabhängig voneinander eine Bindung, C₁₋₈-Alkylen, C₂₋₈-Alkenylen oder C₂₋₈-Alkinylen darstellen, oder abwesend sind;
T1, T2, T3 und T4 unabhängig voneinander
(a) eine Bindung darstellen, oder abwesend sind, oder eine der Gruppen
(b) -CH(OH)-,
(c) -CH(OR⁶)-,
(d) -CH(NR⁵R⁶)-,
(e) -CO-,
(f)-CR⁷R⁸-,
(g) -O- oder -NR⁶⁻,
(h) -S(O)₀₋₂-,
(I) -SO₂NR⁶-,
(j) -NR⁶SO₂-,
(k) -CONR⁶-,
(I) -NR⁶CO-,
(m) -O-CO-,
(n) -CO-O-,
(o) -O-CO-O-,
(p) -O-CO-NR⁶-,
(q) -N(R⁶)-CO-N(R⁶)-,
(r) -N(R⁶)-CO-O-,
(s) Pyrrolidinylen, Piperidinylen oder Piperazinylen, und
(t) -C(R¹¹)(R¹²)- darstellen,
wobei die von (b)-(t) ausgehenden Bindungen zu einem gesättigten oder aromatischen C-Atom der benachbarten Gruppe führen, falls die Bindung von einem Heteroatom ausgeht, und wobei nicht mehr als zwei Gruppen (b)-(f), drei Gruppen (g)-(h) und eine Gruppe (i)-(t) anwesend sind;
R³ Wasserstoff, C₁₋₆-Alkyl, C₂₋₆-Alkenyl, C₁₋₆-Alkoxy, Hydroxy-C₁₋₆-alkyl, C₁₋₆-Alkoxy-C₁₋₆-alkyl oder Benzyl ist;
R⁵ und R⁶ Wasserstoff, C₁₋₆-Alkyl oder Acyl, oder gemeinsam mit dem N-Atom, an das sie gebunden sind, einen 5- oder 6-gliedrigen heterocyclischen Ring, der ein zusätzliches N-, O-oder S-Atom enthalten kann;
R⁷und R⁸ gemeinsam mit dem C-Atom, an das sie gebunden sind, einen 3-7-gliedrigen Ring darstellen, der ein oder zwei -O- oder -S- Atome enthalten kann;
R⁹ Wasserstoff, C₁₋₆-Alkyl, C₃₋₈-Cycloalkyl, Acyl oder Arylalkyl ist;
R¹¹ Wasserstoff oder C₁₋₆-Alkyl ist;
R¹² Wasserstoff oder C₁₋₆-Alkyl ist;
U Wasserstoff, C₁₋₆-alkyl, Cycloalkyl, Cyano, Aryl oder Heterocyclyl;
Q Ethylen oder abwesend ist (Formeln (I+IA)) und Ethylen oder Methylen ist (Formeln (II+IIA));
X eine Gruppe >CH0R⁹, >CO oder >CH-R¹¹ ist;
Z C₁₋₆-Alkylen , -CH-R¹¹-CO-NR⁹-, -O-, -NR⁹-, -Alk-O- , -Alk-NR⁹- oder abwesend ist; und wobei, falls Z -O- darstellt, X >CHR¹¹ ist und entweder R² einen Substituenten L1-T1-L2-T2-L3-T3-L4-T4-L5-U enthält oder R³ ein wie oben definierter, von Wasserstoff verschiedener Substituent ist;
und pharmazeutisch anwendbare Salze davon.

Weiterhin sind Verbindungen der Formeln (I), (IA), (II) und (IIA) bevorzugt, in denen Q abwesend ist. X ist vorzugsweise -CH₂- oder -CO-. Z ist vorzugsweise -O-, -Alk-O-, -N-(C₃₋₈₋Cycloalkyl)-C₁₋₆-alkylen- oder abwesend.

Bevorzugte Reste R¹ sind Phenyl und durch C₁₋₆-Alkyl, C₁₋₆-Alkoxy, Halogen, Hydroxy, Hydroxy-C₁₋₆-alkoxy, C₁₋₆-Alkoxy-C₁₋₆-alkoxy, C₁₋₆-Alkylsulfinyl, Carbamoyl, Cyano, Trifluormethyl, Trifluormethoxy, Carboxy, C₁₋₆-Alkoxy-carbonyl, C₁₋₆-Alkoxy-C₁₋₆-alkoxy-C₁₋₆₋alkyl, C₁₋₆-Alkylcarbonylamino, C₁₋₆-Alkylcarbonylamino-C₁₋₆-alkyl, C₁₋₆-Alkylcarbonylamino-C₁₋₆-alkoxy, (N-C₁₋₆-Alkyl)-C₁₋₆-alkylcarbonylamino-C₁₋₆-alkyl, (N-C₁₋₆-Alkyl)-C₁₋₆-alkylcarbonylamino-C₁₋₆-alkoxy, C₃₋₆-Cycloalkylcarbonylamino-C₁₋₆-alkyl, C₃₋₆-Cycloalkylcarbonylamino-C₁₋₆-alkoxy,
C₁₋₆-Alkoxy-C₁₋₆-alkyl, Hydroxy-C₁₋₆-alkyl, Hydroxy-C₁₋₆-alkoxy-C₁₋₆-alkyl, Hydroxy-C₁₋₆-alkoxy-C₁₋₆-alkoxy, C₁₋₆-Alkoxycarbonylamino-C₁₋₆-alkyl, C₁₋₆-Alkoxycarbonylamino-C₁₋₆-alkoxy, C₁₋₆₋Alkylaminocarbonylamino-C₁₋₆-alkyl, C₁₋₆-Alkylaminocarbonylamino-C₁₋₆-alkoxy, C₁₋₆-Alkylaminocarbonyl-C₁₋₆-alkyl, C₁₋₆-Alkylaminocarbonyl-C₁₋₆-alkoxy, C₁₋₆-Alkylaminocarbonyl-C₁₋₆₋alkoxy-C₁₋₆-alkyl, Di-C₁₋₆-alkylaminocarbonyl-C₁₋₆-alkyl, Di-C₁₋₆-alkylaminocarbonyl-C₁₋₆₋alkoxy, C₁₋₆-Alkylcarbonyloxy-C₁₋₆-alkyl, C₁₋₆-Alkylcarbonyloxy-C₁₋₆-alkoxy, Cyano-C₁₋₆-alkyl, Cyano-C₁₋₆-alkoxy, 2-Oxo-oxazolidinyl-C₁₋₆-alkyl, 2-Oxo-oxazolidinyl-C₁₋₆-alkoxy, C₁₋₆-Alkoxycarbonyl-C₁₋₆-alkyl, C₁₋₆-Alkoxycarbonyl-C₁₋₆-alkoxy, C₁₋₆-Alkylsulfonylamino-C₁₋₆-alkyl, C₁₋₆₋Alkylsulfonylamino-C₁₋₆-alkoxy, (N-C₁₋₆-Alkyl)-C₁₋₆-alkylsulfonylamino-C₁₋₆-alkyl, (N-C₁₋₆-Alkyl)-C₁₋₆-alkylsulfonylamino-C₁₋₆-alkoxy, C₁₋₆-Alkylamino-C₁₋₆-alkyl, C₁₋₆-Alkylamino-C₁₋₆-alkoxy, Di-C₁₋₆-alkylamino-C₁₋₆-alkyl, Di-C₁₋₆-alkylamino-C₁₋₆-alkoxy, C₁₋₆-Alkylsulfonyl-C₁₋₆-alkyl, C₁₋₆₋Alkylsulfonyl-C₁₋₆-alkoxy, Carboxy-C₁₋₆-alkyl, Carboxy-C₁₋₆-alkoxy, Carboxy-C₁₋₆-alkoxy-C₁₋₆₋alkyl, C₁₋₆-Alkoxy-C₁₋₆-alkyl-carbonyl, Acyl-C₁₋₆-alkoxy-C₁₋₆-alkyl, (N-C₁₋₆-Alkyl)-C₁₋₆-alkoxycarbonylamino, (N-Hydroxy)-C₁₋₆-alkylaminocarbonyl-C₁₋₆-alkyl, (N-Hydroxy)-C₁₋₆₋alkylaminocarbonyl-C₁₋₆-alkoxy, (N-Hydroxy)-aminocarbonyl-C₁₋₆-alkyl, (N-Hydroxy)-aminocarbonyl-C₁₋₆-alkoxy, C₁₋₆-Alkoxy-aminocarbonyl-C₁₋₆-alkyl, 6-Alkoxy-aminocarbonyl-C₁₋₆-alkoxy, (N-C₁₋₆-alkoxy)-C₁₋₆-alkylaminocarbonyl-C₁₋₆-alkyl, (N-C₁₋₆-alkOxy)-C₁₋₆₋alkylaminocarbonyl-C₁₋₆-alkoxy, (N-Acyl)-C₁₋₆-alkoxy-C₁₋₆-alkylamino, C₁₋₆-Alkoxy-C₁₋₆-alkyl-carbamoyl, (N-C₁₋₆-Alkyl)-C₁₋₆-alkoxy-C₁₋₆-alkyl-carbamoyl, C₁₋₆-Alkoxy-C₁₋₆-alkyl-carbonyl, C₁₋₆-Alkoxy-C₁₋₆-alkyl-carbonylamino, (N-C₁₋₆-alkyl)-C₁₋₆-Alkoxy-C₁₋₆-alkyl-carbonylamino, 1-C₁₋₆-Alkoxy-C₁₋₆-alkyl-imidazol-2-yl, 1-C₁₋₆-Alkoxy-C₁₋₆-alkyl-tetrazol-5-yl, 5-C₁₋₆-Alkoxy-C₁₋₆₋alkyl-tetrazol-1-yl, 2-C₁₋₆-Alkoxy-C₁₋₆-alkyl-4-oxo-imidazol-1-yl, Carbamoyl-C₁₋₆-alkyl, Carbamoyl-C₁₋₆-alkoxy, C₁₋₆-Alkyl-carbamoyl, Di-C₁₋₆-alkyl-carbamoyl, C₁₋₆-Alkyl-sulfonyl, Piperidinoalkyl, Piperidinoalkoxy, Piperidinoalkoxyalkyl, Morpholinoalkyl, Morpholinoalkoxy, Morpholinoalkoxyalkyl, Piperazinoalkyl, Piperazinoalkoxy, Piperazinoalkoxyalkyl, [1,2,4]-Triazol-1-yl-alkyl, [1,2,4]-Triazol-1-yl-alkoxy, [1,2,4]-Triazol-4-yl-alkyl, [1,2,4]-Triazol-4-yl-alkoxy, [1,2,4]-Oxadiazol-5-yl-alkyl, [1,2,4]-Oxadiazol-5-yl-alkoxy, 3-Methyl-[1,2,4]-oxadiazol-5-yl-alkyl, 3-Methyl-[1,2,4]-oxadiazol-5-yl-alkoxy, 5-Methyl-[1,2,4]-oxadiazol-3-yl-alkyl, 5-Methyl-[1,2,4]-oxadiazol-3-yl-alkoxy, Tetrazol-1-yl-alkyl, Tetrazol-1-yl-alkoxy, Tetrazol-2-yl-alkyl, Tetrazol-2-yl-alkoxy, Tetrazol-5-yl-alkyl, Tetrazol-5-yl-alkoxy, 5-Methyl-tetrazol-1-yl-alkyl, 5-Methyl-tetrazol-1-yl-alkoxy, Thiazol-4-yl-alkyl, Thiazol-4-yl-alkoxy, Oxazol-4-yl-alkyl, Oxazol-4-yl-alkoxy, 2-Oxo-pyrrolidinyl-alkyl, 2-Oxo-pyrrolidinyl-alkoxy, Imidazolyl-alkyl, Imidazolyl-alkoxy, 2-Methyl-imidazolyl-alkyl, 2-Methyl-imidazolyl-alkoxy, N-Methylpiperazinoalkyl, N-Methylpiperazinoalkoxy, N-Methylpiperazinoalkoxyalkyl, Pyrrolidinyl, Piperidinyl, Piperazinyl, Pyrrolyl, 4-Methylpiperazinyl, Morpholinyl, Thiomorpholinyl, 2-Hydroxymethylpyrrolidinyl, 3-Hydroxypyrrolidinyl, 3,4-Dihydroxypyrrolidinyl, 3-Acetamidomethylpyrrolidinyl, 3-C₁₋₆-Alkoxy-C₁₋₆-alkyl-pyrrolidinyl, 4-Hydroxypiperidinyl, 4-Oxopiperidinyl, 3,5-Dimethylmorpholinyl, 4,4-Dioxothiomorpholinyl, 4-Oxothiomorpholinyl, 2,6-Dimethylmorpholinyl, 2-Oxo-imidazolidinyl, 2-Oxo-oxazolidinyl, 2-Oxo-pyrrolidinyl, 2-Oxo-[1,3]oxazinyl oder 2-Oxo-tetrahydro-pyrimidinyl substituiertes Phenyl.

Weitere bevorzugte Reste R¹ sind Naphthyl, durch Hydroxy, Oxo, Halogen, Carbamoyl, Carboxy, C₁₋₆-Alkoxy, Hydroxy-C₁₋₆-alkoxy, C₁₋₆-Alkoxy-C₁₋₆-alkoxy, Di-C₁₋₆-Alkylamino, 2,3-Dihydroxypropoxy, 2,3-Dihydroxypropoxy-C₁₋₆-alkoxy, 2,3-Dimethoxypropoxy, Methoxybenzyloxy, Hydroxybenzyloxy, Phenäthyloxy, Methylendioxybenzyloxy, Dioxolanyl-C₁₋₆-alkoxy, Cyclopropyl-C₁₋₆-alkoxy, Hydroxy-C₁₋₆-alkoxy, Pyridyl-carbamoyloxy-C₁₋₆-alkoxy, 3-Morpholino-2-hydroxypropoxy, Benzyloxy-C₁₋₆-alkoxy, Picolyloxy, C₁₋₆-Alkoxy-carbonyl, C₁₋₆-Alkoxy-C₁₋₆-alkoxy-C₁₋₆-alkyl, C₁₋₆-Alkylcarbonylamino, C₁₋₆-Alkylcarbonylamino-C₁₋₆₋alkyl, C₁₋₆-Alkylcarbonylamino-C₁₋₆-alkoxy, (N-C₁₋₆-Alkyl)-C₁₋₆-alkylcarbonylamino-C₁₋₆-alkyl, (N-C₁₋₆-Alkyl)-C₁₋₆-alkylcarbonylamino-C₁₋₆-alkoxy, C₃₋₆-Cycloalkylcarbonylamino-C₁₋₆-alkyl, C₃₋₆-Cycloalkylcarbonylamino-C₁₋₆-alkoxy, C₁₋₆-Alkoxy-C₁₋₆-alkyl, Hydroxy-C₁₋₆-alkyl, Hydroxy-C₁₋₆-alkoxy-C₁₋₆-alkyl, Hydroxy-C₁₋₆-alkoxy-C₁₋₆-alkoxy, C₁₋₆-Alkoxycarbonylamino-C₁₋₈-alkyl, C₁₋₆-Alkoxycarbonylamino-C₁₋₆-alkoxy, C₁₋₆-Alkylaminocarbonylamino-C₁₋₆-alkyl, C₁₋₆₋Alkylaminocarbonylamino-C₁₋₆-alkoxy, C₁₋₆-Alkylaminocarbonyl-C₁₋₆-alkyl, C₁₋₆-Alkylaminocarbonyl-C₁₋₆-alkoxy, C₁₋₆-Alkylaminocarbonyl-C₁₋₆-alkoxy-C₁₋₆-alkyl, Di-C₁₋₆-alkylaminocarbonyl-C₁₋₆-alkyl, Di-C₁₋₆-alkylaminocarbonyl-C₁₋₆-alkoxy, C₁₋₆-Alkylcarbonyloxy-C₁₋₆-alkyl, C₁₋₆-Alkylcarbonyloxy-C₁₋₆-alkoxy, Cyano-C₁₋₆-alkyl, Cyano-C₁₋₆-alkoxy, 2-Oxo-oxazolidinyl-C₁₋₆-alkyl, 2-Oxo-oxazolidinyl-C₁₋₆-alkoxy, C₁₋₆-Alkoxycarbonyl-C₁₋₆-alkyl, C₁₋₆-Alkoxycarbonyl-C₁₋₆-alkoxy, C₁₋₆-Alkylsulfonylamino-C₁₋₆-alkyl, C₁₋₆-Alkylsulfonylamino-C₁₋₆-alkoxy, (N-C₁₋₆₋Alkyl)-C₁₋₆-alkylsulfonylamino-C₁₋₆-alkyl, (N-C₁₋₆-Alkyl)-C₁₋₆-alkylsulfonylamino-C₁₋₆-alkoxy, C₁₋₆-Alkylamino-C₁₋₆-alkyl, C₁₋₆-Alkylamino-C₁₋₆-alkoxy, Di-C₁₋₆-alkylamino-C₁₋₆-alkyl, Di-C₁₋₆₋alkylamino-C₁₋₆-alkoxy, C₁₋₆-Alkylsulfonyl-C₁₋₆-alkyl, C₁₋₆-Alkylsulfonyl-C₁₋₆-alkoxy, Carboxy-C₁₋₆-alkyl, Carboxy-C₁₋₆-alkoxy, Carboxy-C₁₋₆-alkoxy-C₁₋₆-alkyl, C₁₋₆-Alkoxy-C₁₋₆-alkylcarbonyl, Acyl-C₁₋₆-alkoxy-C₁₋₆-alkyl, (N-C₁₋₆-Alkyl)-C₁₋₆-alkoxy-carbonylamino, (N-Hydroxy)-C₁₋₆-alkylaminocarbonyl-C₁₋₆-alkyl, (N-Hydroxy)-C₁₋₆-alkylaminocarbonyl-C₁₋₆₋alkoxy, (N-Hydroxy)-aminocarbonyl-C₁₋₆-alkyl, (N-Hydroxy)-aminocarbonyl-C₁₋₆-alkoxy, C₁₋₆₋Alkoxy-aminocarbonyl-C₁₋₆-alkyl, 6-Alkoxy-aminocarbonyl-C₁₋₆-alkoxy, (N-C₁₋₆-alkoxy)-C₁₋₆₋alkylaminocarbonyl-C₁₋₆-alkyl, (N-C₁₋₆-alkoxy)-C₁₋₆-alkylaminocarbonyl-C₁₋₆-alkoxy, (N-Acyl)-C₁₋₆-alkoxy-C₁₋₆-alkylamino, C₁₋₆-Alkoxy-C₁₋₆-alkyl-carbamoyl, (N-C₁₋₆-Alkyl)-C₁₋₆-alkoxy-C₁₋₆₋alkyl-carbamoyl, C₁₋₆-Alkoxy-C₁₋₆-alkyl-carbonyl, C₁₋₆-Alkoxy-C₁₋₆-alkyl-carbonylamino, (N-C₁₋₆-alkyl)-C₁₋₆-Alkoxy-C₁₋₆-alkyl-carbonylamino, 1-C₁₋₆-Alkoxy-C₁₋₆-alkyl-imidazol-2-yl, 1-C₁₋₆-Alkoxy-C₁₋₆-alkyl-tetrazol-5-yl, 5-C₁₋₆-Alkoxy-C₁₋₆-alkyl-tetrazol-1-yl, 2-C₁₋₆-Alkoxy-C₁₋₆₋alkyl-4-oxo-imidazol-1-yl, Carbamoyl-C₁₋₆-alkyl, Carbamoyl-C₁₋₆-alkoxy, C₁₋₆-Alkyl-carbamoyl, Di-C₁₋₆-alkyl-carbamoyl, C₁₋₆-Alkyl-sulfonyl, Piperidinoalkyl, Piperidinoalkoxy, Piperidinoalkoxyalkyl, Morpholinoalkyl, Morpholinoalkoxy, Morpholinoalkoxyalkyl, Piperazinoalkyl, Piperazinoalkoxy, Piperazinoalkoxyalkyl, [1,2,4]-Triazol-1-yl-alkyl, [1,2,4]-Triazol-1-yl-alkoxy, [1,2,4]-Triazol-4-yl-alkyl, [1,2,4]-Triazol-4-yl-alkoxy, [1,2,4]-Oxadiazol-5-yl-alkyl, [1,2,4]-Oxadiazol-5-yl-alkoxy, 3-Methyl-[1,2,4]-oxadiazol-5-yl-alkyl, 3-Methyl-[1,2,4]-oxadiazol-5-yl-alkoxy, 5-Methyl-[1,2,4]-oxadiazol-3-yl-alkyl, 5-Methyl-[1,2,4]-oxadiazol-3-yl-alkoxy, Tetrazol-1-yl-alkyl, Tetrazol-1-yl-alkoxy, Tetrazol-2-yl-alkyl, Tetrazol-2-yl-alkoxy, Tetrazol-5-yl-alkyl, Tetrazol-5-yl-alkoxy, 5-Methyl-tetrazol-1-yl-alkyl, 5-Methyl-tetrazol-1-yl-alkoxy, Thiazol-4-yl-alkyl, Thiazol-4-yl-alkoxy, Oxazol-4-yl-alkyl, Oxazol-4-yl-alkoxy, 2-Oxo-pyrrolidinyl-alkyl, 2-Oxo-pyrrolidinyl-alkoxy, Imidazolyl-alkyl, Imidazolyl-alkoxy, 2-Methyl-imidazolyl-alkyl, 2-Methyl-imidazolyl-alkoxy, N-Methylpiperazinoalkyl,
N-Methylpiperazinoalkoxy, N-Methylpiperazinoalkoxyalkyl, Pyrrolidinyl, Piperidinyl, Piperazinyl, Pyrrolyl, 4-Methylpiperazinyl, Morpholinyl, Thiomorpholinyl, 2-Hydroxymethylpyrrolidinyl, 3-Hydroxypyrrolidinyl, 3,4-Dihydroxypyrrolidinyl, 3-Acetamidomethylpyrrolidinyl, 3-C₁₋₆-Alkoxy-C₁₋₆-alkyl-pyrrolidinyl, 4-Hydroxypiperidinyl, 4-Oxopiperidinyl, 3,5-Dimethylmorpholinyl, 4,4-Dioxothiomorpholinyl, 4-Oxothiomorpholinyl, 2,6-Dimethylmorpholinyl, 2-Oxo-imidazolidinyl, 2-Oxo-oxazolidinyl, 2-Oxo-pyrrolidinyl, 2-Oxo-[1,3]oxazinyl oder 2-Oxo-tetrahydro-pyrimidinyl substituiertes Naphthyl; Tetrahydronaphthyl oder durch Methyl substituiertes Tetrahydronaphthyl und Indanyl.

Eine Gruppe besonders bevorzugter Reste R¹ umfasst die oben genannten substituierten Phenyl- und Naphthylreste, sowie Tetrahydronaphthyl und durch Methyl substituiertes Tetrahydronaphthyl.

Ebenfalls bevorzugte Reste R¹ sind Pyridyl, Benzimidazolyl, Di-C₁₋₆-alkoxypyrimidinyl, 2- und 5-Benzo[b]thienyl, 6- und 7-Chinolyl, 6- und 7-Isochinolyl, 6- und 7-Tetrahydrochinolyl, 6- und 7-Tetrahydroisochinolyl, 6-Chinoxalinyl, 6- und 7-Chinazolinyl, Indolyl, Dihydro-3H-benzo[1,4]oxazinyl, 3-Oxo-4H-benzo[1,4]oxazinyl, 2-Oxo-benzooxazolyl, 2-Oxo-1,3-dihydroindolyl, 2,3-Dihydroindolyl, Indazolyl oder Benzofuranyl sowie durch Hydroxy, Oxo, Halogen, Carbamoyl, Carboxy, C₁₋₆-Alkoxy, Hydroxy-C₁₋₆-alkoxy, C₁₋₆-Alkoxy-C₁₋₆-alkoxy, Di-C₁₋₆-Alkylamino, 2,3-Dihydroxypropoxy, 2,3-Dihydroxypropoxy-C₁₋₆-alkoxy, 2,3-Dimethoxypropoxy, Methoxybenzyloxy, Hydroxybenzyloxy, Phenäthyloxy, Methylendioxybenzyloxy, Dioxolanyl-C₁₋₆-alkoxy, Cyclopropyl-C₁₋₆-alkoxy, Hydroxy-C₁₋₆₋alkoxy, Pyridyl-carbamoyloxy-C₁₋₆-alkoxy, 3-Morpholino-2-hydroxypropoxy, Benzyloxy-C₁₋₆₋alkoxy, Picolyloxy, C₁₋₆-Alkoxy-carbonyl C₁₋₆-Alkoxy-C₁₋₆-alkoxy-C₁₋₆-alkyl, C₁₋₆-Alkylcarbonylamino, C₁₋₆-Alkylcarbonylamino-C₁₋₆-alkyl, C₁₋₆-Alkylcarbonylamino-C₁₋₆₋alkoxy, (N-C₁₋₆-Alkyl)-C₁₋₆-alkylcarbonylamino-C₁₋₆-alkyl, (N-C₁₋₆-Alkyl)-C₁₋₆₋alkylcarbonylamino-C₁₋₆-alkoxy, C₃₋₆-Cycloalkylcarbonylamino-C₁₋₆-alkyl, C₃₋₆-Cycloalkylcarbonylamino-C₁₋₆-alkoxy, C₁₋₆-Alkoxy-C₁₋₆-alkyl, Hydroxy-C₁₋₆-alkyl, Hydroxy-C₁₋₆-alkoxy-C₁₋₆-alkyl, Hydroxy-C₁₋₆-alkoxy-C₁₋₆-alkoxy, C₁₋₆-Alkoxycarbonylamino-C₁₋₆-alkyl, C₁₋₆-Alkoxycarbonylamino-C₁₋₆-alkoxy, C₁₋₆-Alkylaminocarbonylamino-C₁₋₆-alkyl, C₁₋₆-Alkylaminocarbonylamino-C₁₋₆-alkoxy, C₁₋₆-Alkylaminocarbonyl-C₁₋₆-alkyl, C₁₋₆-Alkylaminocarbonyl-C₁₋₆-alkoxy, C₁₋₆-Alkylaminocarbonyl-C₁₋₆-alkoxy-C₁₋₆-alkyl, Di-C₁₋₆₋alkylaminocarbonyl-C₁₋₆-alkyl, Di-C₁₋₆-alkylaminocarbonyl-C₁₋₆-alkoxy, C₁₋₆-Alkylcarbonyloxy-C₁₋₆-alkyl, C₁₋₆-Alkylcarbonyloxy-C₁₋₆-alkoxy, Cyano-C₁₋₆-alkyl, Cyano-C₁₋₆-alkoxy, 2-Oxo-oxazolidinyl-C₁₋₆-alkyl, 2-Oxo-oxazolidinyl-C₁₋₆-alkoxy, C₁₋₆-Alkoxycarbonyl-C₁₋₆-alkyl, C₁₋₆-Alkoxycarbonyl-C₁₋₆-alkoxy, C₁₋₆-Alkylsulfonylamino-C₁₋₆-alkyl, C₁₋₆-Alkylsulfonylamino-C₁₋₆-alkoxy, (N-C₁₋₆-Alkyl)-C₁₋₆-alkylsulfonylamino-C₁₋₆-alkyl, (N-C₁₋₆-Alkyl)-C₁₋₆₋alkylsulfonylamino-C₁₋₆-alkoxy, C₁₋₆-Alkylamino-C₁₋₆-alkyl, C₁₋₆-Alkylamino-C₁₋₆-alkoxy, Di-C₁₋₆-alkylamino-C₁₋₆-alkyl, Di-C₁₋₆-alkylamino-C₁₋₆-alkoxy, C₁₋₆-Alkylsulfonyl-C₁₋₆-alkyl, C₁₋₆₋Alkylsulfonyl-C₁₋₆-alkoxy, Carboxy-C₁₋₆-alkyl, Carboxy-C₁₋₆-alkoxy, Carboxy-C₁₋₆-alkoxy-C₁₋₆₋alkyl, C₁₋₆-Alkoxy-C₁₋₆-alkyl-carbonyl, Acyl-C₁₋₆-alkoxy-C₁₋₆-alkyl, (N-C₁₋₆-Alkyl)-C₁₋₆-alkoxycarbonylamino, (N-Hydroxy)-C₁₋₆-alkylaminocarbonyl-C₁₋₆-alkyl, (N-Hydroxy)-C₁₋₆₋alkylaminocarbonyl-C₁₋₆-alkoxy, (N-Hydroxy)-aminocarbonyl-C₁₋₆-alkyl, (N-Hydroxy)-aminocarbonyl-C₁₋₆-alkoxy, C₁₋₆-Alkoxy-aminocarbonyl-C₁₋₆-alkyl, 6-Alkoxy-aminocarbonyl-C₁₋₆-alkoxy, (N-C₁₋₆-alkoxy)-C₁₋₆-alkylaminocarbonyl-C₁₋₆-alkyl, (N-C₁₋₆-alkoxy)-C₁₋₆₋alkylaminocarbonyl-C₁₋₆-alkoxy, (N-Acyl)-C₁₋₆-alkoxy-C₁₋₆-alkylamino, C₁₋₆-Alkoxy-C₁₋₆-alkyl-carbamoyl, (N-C₁₋₆-Alkyl)-C₁₋₆-alkoxy-C₁₋₆-alkyl-carbamoyl, C₁₋₆-Alkoxy-C₁₋₆-alkyl-carbonyl, C₁₋₆-Alkoxy-C₁₋₆-alkyl-carbonylamino, (N-C₁₋₆-alkyl)-C₁₋₆-Alkoxy-C₁₋₆-alkyl-carbonylamino, 1-C₁₋₆-Alkoxy-C₁₋₆-alkyl-imidazol-2-yl, 1-C₁₋₆-Alkoxy-C₁₋₆-alkyl-tetrazol-5-yl, 5-C₁₋₆-Alkoxy-C₁₋₆₋alkyl-tetrazol-1-yl, 2-C₁₋₆-Alkoxy-C₁₋₆-alkyl-4-oxo-imidazol-1-yl, Carbamoyl-C₁₋₆-alkyl, Carbamoyl-C₁₋₆-alkoxy, C₁₋₆-Alkyl-carbamoyl, Di-C₁₋₆-alkyl-carbamoyl, C₁₋₆-Alkyl-sulfonyl, Piperidinoalkyl, Piperidinoalkoxy, Piperidinoalkoxyalkyl, Morpholinoalkyl, Morpholinoalkoxy, Morpholinoalkoxyalkyl, Piperazinoalkyl, Piperazinoalkoxy, Piperazinoalkoxyalkyl, [1,2,4]-Triazol-1-yl-alkyl, [1,2,4]-Triazol-1-yl-alkoxy, [1,2,4]-Triazol-4-yl-alkyl, [1,2,4]-Triazol-4-yl-alkoxy, [1,2,4]-Oxadiazol-5-yl-alkyl, [1,2,4]-Oxadiazol-5-yl-alkoxy, 3-Methyl-[1,2,4]-oxadiazol-5-yl-alkyl, 3-Methyl-[1,2,4]-oxadiazol-5-yl-alkoxy, 5-Methyl-[1,2,4]-oxadiazol-3-yl-alkyl, 5-Methyl-[1,2,4]-oxadiazol-3-yl-alkoxy, Tetrazol-1-yl-alkyl, Tetrazol-1-yl-alkoxy, Tetrazol-2-yl-alkyl, Tetrazol-2-yl-alkoxy, Tetrazol-5-yl-alkyl, Tetrazol-5-yl-alkoxy, 5-Methyl-tetrazol-1-yl-alkyl, 5-Methyl-tetrazol-1-yl-alkoxy, Thiazol-4-yl-alkyl, Thiazol-4-yl-alkoxy, Oxazol-4-yl-alkyl, Oxazol-4-yl-alkoxy, 2-Oxo-pyrrolidinyl-alkyl, 2-Oxo-pyrrolidinyl-alkoxy, Imidazolyl-alkyl, Imidazolyl-alkoxy, 2-Methyl-imidazolyl-alkyl, 2-Methyl-imidazolyl-alkoxy, N-Methylpiperazinoalkyl, N-Methylpiperazinoalkoxy, N-Methylpiperazinoalkoxyalkyl, Pyrrolidinyl, Piperidinyl, Piperazinyl, Pyrrolyl, 4-Methylpiperazinyl, Morpholinyl, Thiomorpholinyl, 2-Hydroxymethylpyrrolidinyl, 3-Hydroxypyrrolidinyl, 3,4-Dihydroxypyrrolidinyl, 3-Acetamidomethylpyrrolidinyl, 3-C₁₋₆-Alkoxy-C₁₋₆-alkyl-pyrrolidinyl, 4-Hydroxypiperidinyl, 4-Oxopiperidinyl, 3,5-Dimethylmorpholinyl, 4,4-Dioxothiomorpholinyl, 4-Oxothiomorpholinyl, 2,6-Dimethylmorpholinyl, 2-Oxo-imidazolidinyl, 2-Oxo-oxazolidinyl, 2-Oxo-pyrrolidinyl, 2-Oxo-[1,3]oxazinyl oder 2-Oxo-tetrahydro-pyrimidinyl substituiertes 6- und 7-Chinolyl, 6- und 7-Isochinolyl, 6- und 7-Tetrahydrochinolyl, 6- und 7-Tetrahydroisochinolyl, 6-Chinoxalinyl, 6- und 7-Chinazolinyl, Indolyl, Dihydro-3H-benzo[1,4]oxazinyl, 3-Oxo-4H-benzo[1,4]oxazinyl, 2-Oxo-benzooxazolyl, 2-Oxo-1,3-dihydroindolyl, 2,3-Dihydroindolyl, Indazolyl und Benzofuranyl.

Bevorzugte Reste R² sind Phenyl, durch Halogen, Hydroxy, Cyano, Trifluormethyl, C₁₋₆-Alkyl, Halo-C₁₋₆-alkyl, Hydroxy-C₁₋₆-alkyl, C₁₋₆-Alkoxy-C₁₋₆-alkyl, Cyano-C₁₋₆-alkyl, Carboxy-C₁₋₆₋alkyl, C₁₋₆-Alkanoyloxy-C₁₋₆-alkyl, C₁₋₆-Alkoxycarbonyloxy-C₁₋₆-alkyl, C₁₋₆-Alkoxycarbonyl, C₁₋₆₋Alkoxy oder C₁₋₆-Alkylendioxy substituiertes Phenyl.

Ebenfalls bevorzugte Reste R² sind durch einen Rest L1-T1-L2-T2-L3-T3-L4-T4-L5-U substituiertes Phenyl, wobei L1 und L2 vorzugsweise abwesend oder C₁₋₈-Alkylen sind und L3 abwesend ist und U Wasserstoff, C₁₋₆-Alkyl, Cyclo-C₃₋₆-alkyl, Phenyl-piperidinyl, Phenyl-piperazinyl, Phenyl-pyrrolidinyl, Phenyl, durch C₁₋₆-Alkyl, C₁₋₆-Alkoxy, C₁₋₆-Alkylthio, C₁₋₆₋Alkylsulfinyl, C₁₋₆-Alkylendioxy, Halogen, Benzoyl-C₁₋₆-alkyl, Halogen-C₁₋₆-alkyl, C₁₋₆₋Alkanoyloxy oder Hydroxy substituiertes Phenyl; oder Naphthyl; oder Pyridyl, Thienyl, Pyrazinyl, Triazolyl, Imidazolyl, Phenyl-oxadiazolyl, Thienyl-oxadiazolyl, Furyl-oxadiazolyl, Phenyloxazolyl, Benzthiazolyl, Furyl, Pyrimidinyl, Nitrobenzthiazolyl, Phenyltetrazolyl, Piperidinyl, Tetrahydropyranyl oder Morpholinyl darstellt.

Bei den Gruppen T1-T4 sind die Bedeutungen (a)-(c), (e)-(h), (k)-(n) und (r)-(t) bevorzugt. Beispiele besonders bevorzugter Reste R² sind Phenyl oder durch
2-Benzothiazolyl-thio-C₁₋₆-alkyl,
2-Benzyloxy-3-methoxypropoxy, 2-Benzoyloxy-3-methoxypropoxy, 2,3-Dihydroxypropoxy,
2-Hydroxy-3-benzylamino-propoxy, 2-Hydroxy-3-phenoxypropoxy, 2-Hydroxy-3-phenylthiopropoxy, 2-Methoxy-3-phenoxypropoxy, 2-Methoxy-3-benzyloxypropoxy, 2-Methyl-3-fluor -phenylbutyryloxy-C₁₋₆-alkoxy, 2-Methyl-3-phenoxypropoxy, 2-C₁₋₆-Alkenyloxy-4-phenylbutyl, 3,4,5-Trimethoxyphenyl-oxadiazolyl-C₁₋₆-alkoxy, 6-Nitro-2-benzothiazolyl-thio-C₁₋₆-alkyl, Benzamido-C₁₋₆-alkoxy, Benzamido-C₁₋₆-alkyl, Benzo[1,3]dioxolyloxy-C₁₋₆-alkoxy, Benzoyl-C₁₋₆-alkoxy und Ketale davon, Benzoyl-C₁₋₆-alkyl und Ketale davon, Benzoyl-C₁₋₆-alkyl-aminocarbonyl-C₁₋₆-alkyl, Benzoyl-C₁₋₆-alkoxycarbonyl-C₁₋₆-alkyl, Benzoyl-C₁₋₆-alkyl-aminocarbonyl, Benzoyloxy, Benzoyloxy-C₁₋₆-alkyl-benzoyloxy-C₁₋₆-alkoxy, Benzoyloxy-C₁₋₆-alkoxy, Benzoyloxy-C₁₋₆-alkyl, Benzthiazolylthio-C₁₋₆-alkoxy, Benzthiazolylthio-C₁₋₆alkyl, Benzylcarbamoyl-C₁₋₆-alkoxy, Benzyloxy-C₁₋₆-alkoxycarbonyloxy-C₁₋₆-alkyl, Benzyloxy-C₁₋₆-alkoxy, Benzylthio-C₁₋₆-alkoxy, Carbamoyloxy-C₁₋₆-alkoxy, Carbamoyloxy-C₁₋₆-alkyl, Carboxy-C₁₋₆-alkoxy, Carboxy-C₁₋₆-alkyl, Cyano, Cyano-C₁₋₆-alkoxy, Cyano-C₁₋₆-alkyl, Cyanophenyl-C₁₋₆-alkoxy, Cyclohexylcarbonyloxy-C₁₋₆-alkyl, Cyclohexyloxy-C₁₋₆-alkoxy, Cyclopropylcarbonyloxy-C₁₋₆-alkyl, Dioxolanyl-C₁₋₆-alkoxy, Furyl-oxadiazolyl-C₁₋₆-alkoxy, Furoyloxy-C₁₋₆-alkoxy, Halo-phenoxy-C₁₋₆-alkyl, Halobenzoyl-C₁₋₆-alkoxy, Halobenzoyloxy-C₁₋₆-alkyl, Halobenzoyloxy-C₁₋₆-alkoxy, Halobenzyloxy-C₁₋₆-alkoxy, Halogen, Halogen-C₁₋₆-alkyl, Halophenoxy, Halophenoxy-C₁₋₆-alkoxy, Halophenyl-oxadiazolyl-C₁₋₆-alkoxy, Hydroxy, Hydroxy-benzoyloxy-C₁₋₆-alkyl, Hydroxy-benzoyloxy-C₁₋₆-alkoxy, Hydroxy-C₁₋₆-alkoxy, Hydroxy-C₁₋₆-alkyl, Imidazolylcarbonyloxy-C₁₋₆-alkyl, Methoxybenzoyl-C₁₋₆-alkyl, Methoxybenzyl-oxy-C₁₋₆-alkoxy, Methylendioxybenzoyl-C₁₋₆-alkoxy, Morpholino-C₁₋₆-alkoxy, Morpholinocarbonyloxy-C₁₋₆-alkoxy, Morpholinocarbonyloxy-C₁₋₆-alkyl, N-Methylaminophenyl-carbonyloxy-C₁₋₆-alkyl, N-Methyl-benzylamino-C₁₋₆-alkoxy, N-Methylpyrrolylcarbonyloxy-C₁₋₆-alkoxy, N-C₁₋₆-Alkylbenzamido-C₁₋₆-alkyl, Naphthyl-C₁₋₆-alkoxy, Nicotinoyloxy-C₁₋₆-alkoxy, Nicotinoyloxy-C₁₋₆-alkyl, C₁₋₆-Alkanoylbenzoyloxy-C₁₋₆-alkyl, C₁₋₆-Alkanoyloxy-C₁₋₆-alkoxy, C₁₋₆-Alkanoyloxy-C₁₋₆-alkyl, C₁₋₆-Alkenyl-benzyloxy-C₁₋₆-alkoxy, C₁₋₆-Alkenyloxy, C₁₋₆-Alkenyloxy-benzyloxy-C₁₋₆-alkoxy, C₁₋₆-Alkoxy, C₁₋₆-Alkoxy-benzoyloxy-C₁₋₆-alkyl, C₁₋₆-Alkoxy-carbonyl, C₁₋₆-Alkoxy-C₁₋₆-alkyl, C₁₋₆-Alkoxybenzoylamino-C₁₋₆-alkyl, C₁₋₆-Alkoxybenzylcarbonyloxy-C₁₋₆-alkyl, C₁₋₆-Alkoxy-benzyloxy-C₁₋₆-alkoxy, C₁₋₆-Alkoxy-benzylthio-C₁₋₆-alkoxy, C₁₋₆-Alkoxy-carbonyl-C₁₋₆-alkoxy, C₁₋₆-Alkoxy-carbonyl-C₁₋₆-alkyl, C₁₋₆-Alkoxyphenyl-oxadiazolyl-C₁₋₆-alkoxy, C₁₋₆-Alkoxy-phenyloxy-C₁₋₆-alkoxy, C₁₋₆-Alkyl, C₁₋₆-Alkyl-benzyloxy-C₁₋₆-alkoxy, C₁₋₆-Alkyl-phenoxy-C₁₋₆-alkoxy, C₁₋₆-Alkylendioxy, C₁₋₆-Alkylendioxybenzyloxy-C₁₋₆-alkoxy, C₁₋₆-Alkylsulfonylbenzoyl-C₁₋₆-alkoxy, C₁₋₆-Alkylthiobenzoyloxy-C₁₋₆-alkoxy, C₁₋₆-Alkylthio-benzyloxy-C₁₋₆-alkoxy, Benzoyloxybenzyl-C₁₋₆-alkoxy, Hydroxybenzyl-C₁₋₆-alkoxy, C₁₋₆-Alkoxybenzyl-C₁₋₆-alkoxy, C₁₋₆-Alkoxybenzylcarbonyloxy-C₁₋₆-alkoxy, Phenoxy-benzyloxy-C₁₋₆-alkoxy, Phenoxycarbonyl-C₁₋₆-alkyl, Phenoxy-C₁₋₆-alkenyloxy, Phenoxy-C₁₋₆-alkinyloxy, Phenyl-C₁₋₆-alkanoylamino-C₁₋₆-alkyl, Phenyl-C₁₋₆-alkenyloxy, Phenyl-C₁₋₆-alkoxy, Phenyl-C₁₋₆-alkyl, Phenyl-C₁₋₆-alkylaminocarbonyl, Phenyl-C₁₋₆-alkylcarbonyl-C₁₋₆-alkoxy, Phenyl-C₁₋₆-alkylaminocarbonyl-C₁₋₆-alkyl, Phenylaminocarbonyloxy-C₁₋₆-alkoxy, Phenylaminocarbonyloxy-C₁₋₆-alkyl, Phenyl-hydroxy-C₁₋₆-alkyl, Phenyl-oxadiazolyl-C₁₋₆-alkoxy, Phenyl-oxadiazolyl-C₁₋₆-alkyl, Phenyl-oxazolyl-C₁₋₆-alkoxy, Phenyloxy-C₁₋₆-alkoxy, Phenylsulfamoyl-C₁₋₆-alkyl, Phenylsulfinyl-C₁₋₆-alkyl, Phenylsulfonyl-C₁₋₆-alkoxy, Phenylsulfonyl-C₁₋₆-alkyl, Phenyltetrazolyl-thio-C₁₋₆-alkyl, Phenylthio-C₁₋₆-alkoxy, Phenylthio-C₁₋₆-alkyl, Pyrazinylcarbonyloxy-C₁₋₆-alkyl, Pyridylaminocarbonyloxy-C₁₋₆-alkoxy, Pyridylaminocarbonyloxy-C₁₋₆-alkyl, Pyridylcarbamoyloxy, Pyridyl-C₁₋₆-alkoxy-C₁₋₆-alkoxy, Pyridyl-C₁₋₆-alkoxy-C₁₋₆-alkyl, Pyridyl-oxadiazolyl-C₁₋₆-alkoxy, Pyridylthio-C₁₋₆-alkyl, Pyrimidinyloxy-C₁₋₆-alkoxy, Pyrimidinylthio-C₁₋₆-alkyl, Thienoyloxy-C₁₋₆-alkoxy, Thienoyloxy-C₁₋₆-alkyl, Thienyl-oxadiazolyl-C₁₋₆-alkoxy, Triazolyl-C₁₋₆-alkoxy, Trifluormethylbenzyloxy-C₁₋₆-alkoxy, oder Trifluormethyl-substituiertes Phenyl.

Beispiele besonders bevorzugter Reste R¹ sind Phenyl, 3,4-Dihydro-2H-benzo[1,4]oxazin-6-yl und 4H-Benzo[1,4]oxazin-3-on-6-yl, welches durch 1-3 Halogen oder C₁₋₆-Alkoxy-C₁₋₆-alkyl substituiert sein kann.

Beispiele besonders bevorzugter Reste R² ist durch C₁₋₆-Alkoxy, C₁₋₆-Alkoxybenzyloxy-C₁₋₆₋alkoxy oder Halophenoxy-C₁₋₆-alkoxy-substituiertes Phenyl.

Beispiele besonders bevorzugter Reste X sind Methylen und >CO.

Beispiele besonders bevorzugter Reste Z sind O- und -N-(C₃₋₈-Cycloalkyl)-C₁₋₈-alkylen-.

Die Verbindungen der Formel (I) oder (II) können in analoger Weise zu aus der Literatur bekannten Herstellungsverfahren hergestellt werden. Einzelheiten zu den spezifischen Herstellungsvarianten können den Beispielen entnommen werden.

Die Verbindungen der Formel (I) oder (II) können auch in optisch reiner Form dargestellt werden. Die Trennung in Antipoden kann nach an sich bekannten Methoden erfolgen entweder vorzugsweise auf einer synthetisch frühen Stufe durch Salzbildung mit einer optisch aktiven Säure wie zum Beispiel (+)- oder (-)-Mandelsäure und Trennung der diasteromeren Salze durch fraktionierte Kristallisation oder vorzugsweise auf einer eher späten Stufe durch Derivatisierung mit einem chiralen Hilfsbaustein, wie zum Beispiel (+)-oder (-)-Camphansäurechlorid, und Trennung der diastereomeren Produkte durch Chromatographie und/oder Kristallisation und anschließende Spaltung der Bindung zum chiralen Hilfsstoff. Die reinen diastereomeren Salze und Derivate können zur Bestimmung des absoluten Konfiguration des enthaltenen Piperidines mit gängigen spektroskopischen Methoden analysiert werden, wobei die X-Ray Spektroskopie an Einkristallen eine besonders geeignete Methode darstellt.

Eine bevorzugte Methode zur Herstellung optisch reiner Verbindungen der Formel (IA) oder (IIA) besteht im Aufbau des Piperazingrundgerüstes durch Umsetzung eines Oxazolidins mit einem Amin zu einem Piperazindion gemäss dem folgenden beispielhaften Schema:

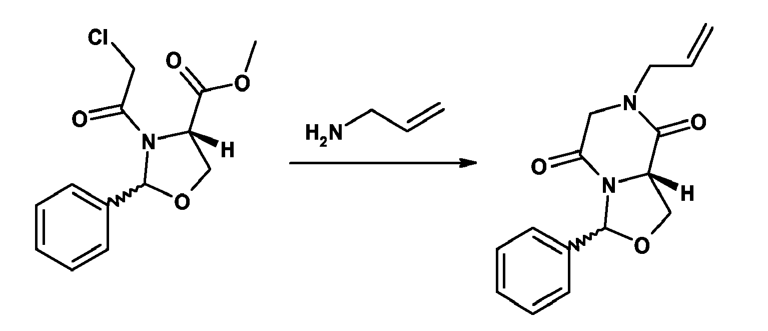

Nähere Einzelheiten und alternative Herstellungsverfahren sind in Tetrahedron Asymmetry 12 (2001), 1293-1302 bzw. dort zitierter Literatur angegeben.

Eine andere bevorzugte Methode zur Herstellung optisch reiner Verbindungen der Formel (IA) oder (IIA) besteht im Aufbau des Piperazingrundgerüstes durch Umsetzung eines substituierten Epoxids mit einem 2-Amino-acetamid, anschliessender Aktivierung des Alkohols und Ringschluss zum Piperazinon gemäss dem folgenden beispielhaften Schema:

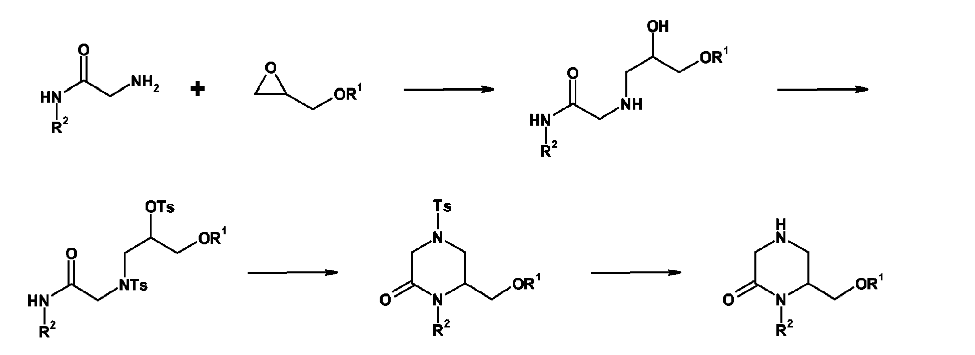

Alternative Herstellungsverfahren sind in Tetrahedron Letters 39 (1998), 7459-7462 bzw. dort zitierter Literatur angegeben.

Prodrug Derivate der vorliegend beschriebenen Verbindungen sind Derivate derselben, die bei *in vivo* Anwendung die ursprüngliche Verbindung durch einen chemischen oder physiologischen Prozess freisetzen. Ein Prodrug kann beispielsweise bei Erreichen eines physiologischen pH-Wertes oder durch enzymatische Konversion in die ursprüngliche Verbindung umgesetzt werden. Prodrug Derivate können zum Beispiel Ester freiverfügbarer Carbonsäuren, S- und O-Acylderivate von Thiolen, Alkoholen oder Phenolen darstellen, wobei die Acylgruppe wie vorliegend definiert wird. Bevorzugt werden pharmazeutisch verwendbare Esterderivate, die durch Solvolyse in physiologischem Medium in die ursprüngliche Carbonsäure umgesetzt werden, wie beispielsweise niedere Alkylester, Zykloalkylester, niedere Alkenylester, Benzylester, mono- oder disubstituierte niedere Alkylester, wie niedere ω-(Amino, Mono- oder Dialkylamino, Carboxy, niedere Alkoxycarbonyl) - Alkylester oder wie niedere α-(Alkanoyloxy, Alkoxycarbonyl oder Diaikyiaminocarbonyl) -Alkylester; als solche werden Pivaloyloxymethylester and ähnliche Ester konventioneller Weise benützt.

Aufgrund der nahen Verwandtschaft zwischen einer freien Verbindung, einem Prodrug Derivat und einer Salzverbindung, umfasst eine bestimmte Verbindung in dieser Erfindung auch dessen Prodrug Derivat und Salzform, sofern diese möglich und angebracht ist.

Die Verbindungen der Formel (I) oder (II), bzw. bevorzugt der Formel (IA) oder (IIA), und ihre pharmazeutisch verwendbaren Salze weisen hemmende Wirkung hinsichtlich Beta-Sekretase, Cathepsin D, Plasmepsin II und/oder HIV-Protease auf.

Die Wirkung von Beta-Sekretase-, Cathepsin D-, Plasmepsin II- und/oder HIV-Protease-Hemmern wird unter anderem experimentell mittels in vitro-Tests nachgewiesen.

Der Protease hemmende Vorgang von chemischen Verbindungen kann anhand von Testsystemen, die auf der Fluoreszenz-Resonanz Energietransfer (FRET) Technologie basiert sind und ein rekombinantes, zum Beispiel durch ein Baculovirus exprimiertes Enzympräparat beigeben, ermittelt werden. Die FRET Technologie erlaubt die Messung der proteolytische Teilung von Peptidsubstraten. Das Prinzip der FRET Technologie beruht auf einem messbaren Energiewechsel, der quantitative von dem Vorliegen einer Peptidsequenz abhängig ist. Das Peptidsubstrat für ein bestimmtes Enzym wird mit zwei terminalen Fluorophoren versehen. Das eine Fluorophor stellt den Fluoreszenz-Donor dar und das andere den Fluoreszenz Akzeptor. Die Distanz dieser beiden Gruppen ist so gewählt, das die lichtinduzierte Donorfluoreszenz vom Akzeptor durch FRET gelöscht wird. Bei proteolytischer Substratspaltung, wird das Donorfluorophor vom Akzeptorfluorophor getrennt, wodurch der Fluoreszenzgehalt des Donors nicht gelöscht wird. Auf diese Weise wird ein schwach fluoreszierendes Peptidsubstrat nach enzymischer Spaltung zu einem hoch fluoreszierendem Peptid. Die Steigerung der Fluoreszenz hängt linear von der Proteolyserate ab.

Das FRET Testsystem wird mit weissen Polysorp-Platten durchgeführt. Das Assaymedium besteht aus einer 50 mM Natriumazetat Pufferlösung von pH 5.0, einer Natriumkonzentration von 392 mM, einer Glyceringehalt von 12.5% und einem BSA-Gehalt von 0.1 %. Die Inkubate setzen sich aus 160 µl Pufferlösung, 10 µl Inhibitoren in DMSO, 10 µl Peptidsubstrat in DMSO und 20 µl Enzympräparatslösung zusammen. Die Inhibitoren werden in einem Konzentrationsbereich von 0.001 pM zu 10 µM getested. Die zugefügte Peptidsubstratmenge ergibt eine Konzentration im Inkubat von 1 µM. Die fluorophoren Donor und Akzeptor markierten Peptidsubstrate werden durch Solidphasenpeptidsynthese (Applied Biosystems) hergestellt. Das beta-Sekretase Peptidsubstrat Rh-Glu-Val-Asn-Leu-Asp-Ala-Glu-Phe-Lys-Quencher wird von Invitrogen, Carlsbad, CA, USA erhalten. Das Cathepsin D Peptidsubstrat mit der Sequenz DABCYL-Pro-Thr-Glu-Phe-Phe-Arg-Leu-OXL wird von der AnaSpec Inc, San Jose, CA, USA erhalten. Das Plasmepsin Peptidsubstrat mit der Sequenz DABCYL-Glu-Arg-Nle-Phe-Leu-Ser-Phe-Pro-OXL wird von der AnaSpec Inc, San Jose, CA, USA erhalten. Das HIV Protease Peptidsubstrat mit der Sequenz DABCYL-His-Lys-Ala-Arg-Val-Leu-Tyr-Glu-Ala-Nle-Ser-EDANS wird von der AnaSpec Inc, San Jose, CA, USA erhalten.
Die rekombinant exprimierten Enzympräparate werden in unterschiedlichen Mengen hinzugegeben. Die Beta-Sekretase Konzentration beträgt 1 unit/ml im Inkubat. Die Cathepsin D Konzentration beträgt 100 ng/ml. Die HIV Protease Konzentration beträgt 500 ng/ml. Die Plasmepsin II Konzbetration im Inkubat ist 50 ng/ml.
Die Reaktionen werden durch Zuführung der Enzymlösung eingeleitet. Der Test wird bei einer Temperatur von 37°C während 30-120 Minuten durchgeführt. Für den Beta-Sekretase Assay beträgt die Inkubationszeit 60 min, für Cathepsin D 120 min, für Plasmepsin II 40 min und für die HIV Protease 40 min. Die Reaktionen werden durch die Zugabe von 20 µl einer 1.0 M Tris Basis Lösung gestoppt. Die enzymatische Substrat zu Produkt Umwandlung wird durch Messung der Fluoreszenz bei 460 nm Wellenlänge gemessen.

### In vitro Enzymhemmungswerte

Für die erfindungsgemässen Verbindungen werden strukturabhängige und enzymabhängige Hemmungsaktivitäten gemessen. Die Enzymhemmungen wurden als IC50 Werte ausgedrückt. Die gemessenen IC50 -Werte zur Inhibierung der Beta-Sekretase belaufen sich zwischen 0.01 pM und 100 nM. Die IC50 -Werte zur Inhibierung von Cathepsin D sind zwischen 0.01 pM und 100 nM. Die IC50-Werte zur Inhibierung von Plasmepsin II liegen zwischen 0.01 pM und 100 nM. Die gemessenen IC50 -Werte zur Inhibierung der HIV-Peptidase liegen zwischen 0.01 pM und 100 nM.

Die Verbindungen der Formel (I) oder (II), bzw. bevorzugt der Formel (IA) oder (IIA), und ihre pharmazeutisch verwendbaren Salze können als Heilmittel, z.B. in Form pharmazeutischer Präparate, Verwendung finden. Die pharmazeutischen Präparate können enteral, wie oral, z.B. in Form von Tabletten, Lacktabletten, Dragees, Hart- und Weichgelatinekapseln, Lösungen, Emulsionen oder Suspensionen, nasal, z.B. in Form von Nasensprays, rektal, z.B. in Form von Suppositorien, oder transdermal, z.B. in Form von Salben oder Pflastern, verabreicht werden. Die Verabreichung kann aber auch parenteral, wie intramuskulär oder intravenös, z.B. in Form von Injektionslösungen, erfolgen.

Zur Herstellung von Tabletten, Lacktabletten, Dragees und Hartgelatinekapseln können die Verbindungen der Formel (I) oder (II), bzw. bevorzugt der Formel (IA) oder (IIA), sowie ihre pharmazeutisch verwendbaren Salze mit pharmazeutisch inerten, anorganischen oder organischen Excipientien verarbeitet werden. Als solche Excipientien kann man z.B. für Tabletten, Dragees und Hartgelatinekapseln, Laktose, Maisstärke oder Derivate davon, Talk, Stearinsäure oder deren Salze etc. verwenden.
Für Weichgelatinekapseln eignen sich als Excipientien z.B. vegetabile Öle, Wachse, Fette, halbfeste und flüssige Polyole etc.
Zur Herstellung von Lösungen und Sirupen eignen sich als Excipientien z.B. Wasser. Polyole, Sacharose, Invertzucker, Glukose etc.
Für Injektionslösungen eignen sich als Excipientien z.B. Wasser, Alkohole, Polyole, Glycerin, vegetabile Öle, Gallensäuren, Lecithin etc.
Für Suppositorien eignen sich als Excipientien z.B. natürliche oder gehärtete Öle, Wachse, Fette, halbflüssige oder flüssige Polyole etc.
Die pharmazeutischen Präparate können daneben noch Konservierungsmittel, Lösungsvermittler, viskositätserhöhende Stoffe, Stabilisierungsmittel, Netzmittel, Emulgiermittel, Süssmittel, Färbemittel, Aromatisierungsmittel, Salze zur Veränderung des osmotischen Druckes, Puffer, Überzugsmittel oder Antioxidantien enthalten. Sie können auch noch andere therapeutisch wertvolle Stoffe enthalten.

Ein weiterer Gegenstand der vorliegenden Erfindung ist die Verwendung der Verbindungen der Formel (I) oder (II), bzw. bevorzugt der Formel (IA) oder (IIA), und ihrer pharmazeutisch verwendbaren Salze bei der Behandlung bzw. Verhütung von zur Prevention, zur Progressionsverzögerung oder zur Behandlung von Alzheimer, Malaria oder HIV.

Die Dosierung kann innerhalb weiter Grenzen variieren und ist natürlich in jedem einzelnen Fall den individuellen Gegebenheiten anzupassen. Im allgemeinen dürfte bei oraler Verabreichung eine Tagesdosis von etwa 3 mg bis etwa 3 g, vorzugsweise etwa 10 mg bis etwa 1 g, z.B. ungefähr 300 mg pro erwachsene Person (70 kg), verteilt auf vorzugsweise 1-3 Einzeldosen, die z.B. gleich gross sein können, angemessen sein, wobei aber die angegebene obere Grenze auch überschritten werden kann, wenn sich dies als angezeigt erweisen sollte, üblicherweise erhalten Kinder eine ihrem Alter und Körpergewicht entsprechend geringere Dosis.

Die folgenden Beispiele erläutern die vorliegende Erfindung. Alle Temperaturen sind in Celsiusgraden, Drucke in mbar angegeben. Falls nicht anders erwähnt finden die Reaktionen bei Raumtemperatur statt. Die Abkürzung "Rf = xx (A)" bedeutet beispielsweise, dass der Rf-Wert xx im Lösungsmittelsystem A ermittelt wird. Das Mengenverhältnis von Lösungsmitteln zueinander ist stets in Volumenanteilen angegeben. Chemische Namen für End- und Zwischenprodukte wurden mit Hilfe des Programmes AutoNom 2000 (Automatic Nomenclature) erzeugt. Falls nicht anders erwähnt ist die absolute Stereochemie der 2-Substituiertenpiperazin-Einheit (2R).

### HPLC-Gradienten auf Hypersil BDS C-18 (5 um); Säule: 4 x 125 mm

I 90% Wasser /10% Acetonitril / 0,1% Trifluoressigsäure zu 0% Wasser / 100% Acetonitril / 0,1% Trifluoressigsäure in 5 Minuten + 2,5 Minuten (1,5 ml/min)
II 95% Wasser / 5% Acetonitril / 0,1 % Trifluoressigsäure zu 0% Wasser /100% Acetonitril / 0,1% Trifluoressigsäure in 40 Minuten (0,8 ml/min)

Es werden die folgenden Abkürzungen verwendet:
- Rf: Verhältnis von Laufstrecke einer Substanz zu Entfernung der Laufmittelfront vom Startpunkt bei Dünnschichtchromatographie
- Rt: Retentionszeit einer Substanz bei HPLC (in Minuten)
- Smp.: Schmelzpunkt (Temperatur)

### Generelle Methode A: (De-Boc 1)

Die Lösung von 1 mMol "Boc-Derivat" und 5 ml Chloroform wird nacheinander mit 15 ml Methanol und 2,5 ml 2N HCl versetzt und während 18 Stunden bei 60 °C gerührt. Das Reaktionsgemisch wird auf Raumtemperatur gekühlt, auf 1 M wässrige Natriumhydrogencarbonat-Lösung (40 ml) gegossen und mit tert.-Butylmethylether (2 x 60 ml) extrahiert. Die organischen Phasen werden mit Sole (1x 60 ml) gewaschen, mit Natriumsulfat getrocknet und eingedampft. Aus dem Rückstand wird mittels Flashchromatographie (SiO₂ 60F) die Titelverbindung erhalten.

### Generelle Methode B: (De-Cbz)

Die Lösung von 1 mMol "Cbz-Derivat" und 15 ml Tetrahydrofuran wird in Gegenwart von 100 - 200 mg Pd/C 10 % während 2 - 4 Stunden bei 15 - 20 °C hydriert. Das Reaktionsgemisch wird klarfiltriert und das Filtrat eingedampft. Aus dem Rückstand wird mittels Flashchromatographie (SiO₂ 60F) die Titelverbindung erhalten.

### Generelle Methode C: (9-BBN Reduktion)

Die Lösung von 1 mMol "Laktam" und 3 ml Tetrahydrofuran wird mit 9-BBN (0,5 M in Tetrahydrofuran) (3,2 - 6,4 equiv.) versetzt und während 1 - 2 Stunden am Rückfluss gerührt (Umsatzkontrolle mit HPLC). Das Reaktionsgemisch wird auf Raumtemperatur gekühlt, mit Ethanolamin (3,2 - 6,4 equiv.) versetzt und eingedampft. Der Rückstand wird in Essigester - Heptan 1:1 (30 ml) bei 0 °C über Nacht verrührt, klarfiltriert und das Filtrat eingedampft. Aus dem Rückstand wird mittels Flashchromatographie (SiO₂ 60F) die Titelverbindung erhalten.

### Generelle Methode D: (De-Boc II)

Eine Lösung von 0,033 mMol "Boc-Derivat" in 1 ml Dichlormethan wird mit 0,3 ml Trifluoressigsäure versetzt. Die Reationsmischung wird während 1 Stunde bei Raumtemperatur gerührt. Das Reaktionsgemisch wird eingedampft und in 5 ml Dichlormethan aufgenommen. Die organische Phase wird mit 5 ml 0.5 M NaOH gewaschen. Die wässrige Phase wird mit Dichlormethan extrahiert (2 x 5 ml). Die vereinigten organischen Phasen werden mit Natriumsulfat getrocknet und eingedampft. Aus dem Rückstand wird mittels Flashchromatographie (SiO₂ 60F) die Titelverbindung erhalten.

### Beispiel 1:

### 6-(1-{4-[3-(2-Methoxy-benzyloxy)-propoxyl-phenyl}-piperazin-2-ylmethoxy)-4-(3-methoxypropyl)-4H-benzo[1,4]oxazin-3-on

Analog Methode A wird aus 0,112 g 4-(4-[3-(2-Methoxy-benzyloxy)-propoxy]-phenyl}-3-[4-(3-methoxy-propyl)-3-oxo-3,4-d ihydro-2H-benzo[1,4]oxazin-6-yloxymethyl]-piperazin-1-carbonsäure tert-butyl ester die Titelverbindung hergestellt.

Die Ausgangsmaterialien werden wie folgt hergestellt:

### a) 4-{4-[3-(2-Methoxy-benzyioxy)-propoxy]-phenyl}-3-[4-(3-methoxv-propyl)-3-oxo-3,4-dihydro-2H-benzo[1,4]oxazin-6-yloxymethyl]-piperazin-1-carbonsäure tert-butyl ester

Eine Mischung von 0,085 g 1-(3-(2-methoxy-benzyloxy)-propoxy)-4-brombenzol, 0,070 g 3-[4-(3-Methoxy-propyl)-3-oxo-3,4-d ihydro-2H-benzo[1,4]oxazin-6-yloxymethyl]-pi perazin-1-carbonsäure tert-butyl ester, 0,023 g Natrium-tert-butylat und 0,008 g Tris(dibenzylidenaceton)dipalladium(0)•Chloroform Komplex wird in einer Schlenkapparatur unter Argon vorgelegt, mit einer Lösung von 0,003 g Tri-tert-butyl-phosphin in 2 ml Toluol (hergestellt in einer Schlenkapparatur unter Argon) versetzt und während 1 Stunde bei 120 °C gerührt. Das Reaktionsgemisch wird auf Raumtemperatur abgekühlt, mit Essigester verdünnt und mit gesättigter wässriger Natriumhydrogencarbonat-Lösung gewaschen. Die organische Phase wird mit Natriumsulfat getrocknet und eingedampft. Aus dem Rückstand wird mittels Flashchromatographie (SiO₂ 60F) die Titelverbindung als braunes Öl erhalten. Rf = 0,44 (EtOAc-Heptan 1:1); Rt = 4,92.

### b) 3-[4-(3-Methoxy-propyl)-3-oxo-3,4-dihydro-2H-benzo[1,4]oxazin-6-yloxymethyl]-piperazin-1-carbonsäure tert-butyl ester

Eine Lösung von 1,43 g 4-Benzyl-3-[4-(3-methoxy-propyl)-3-oxo-3,4-dihydro-2H-benzo[1,4]oxazin-6-yloxymethyl]-piperazin-1-carbonsäure tert-butyl ester in 100 ml Ethanol wird in Gegenwart von 0,145 g Pd/C 10% während 16 Stunden bei Raumtemperatur hydriert. Das Reaktionsgemisch wird klarfiltriert und das Filtrat eingedampft. Aus dem Rückstand wird mittels Flashchromatographie (SiO₂ 60F) die Titelverbindung als gelbliches Öl erhalten.
Rf = 0,63 (Dichlormethan-Methanol 9:1); Rt = 3,28.

### c) 4-Benzyl-3-[4-(3-methoxy-propyl)-3-oxo-3,4-dihydro-2H-benzo[1,4]oxazin-6-yloxymethyl]-piperazin-1-carbonsäure tert-butyl ester

Eine Suspension von 0,041 g Kupfer(I)iodid, 0,079 g [1,10]Phenanthrolin, 0,986 g Cäsiumcarbonat, 0.75 g 6-lod-4-(3-methoxy-propyl)-4H-benzo[1,4]oxazin-3-on und 1,32 g 4-Benzyl-3-hydroxymethyl-piperazin-1-carbonsäure tert-butyl ester in 10 ml Toluol wird während 40 Stunden bei 110 °C in einem verschlossenen Supelcofläschen gerührt. Das Reaktionsgemisch wird auf Raumtemperatur abgekühlt, über Kieselgel filtriert und der Filterkuchen mit Essigester nachgewaschen. Das Filtrat wird eingedampft. Aus dem Rückstand wird mittels Flashchromatographie (SiO₂ 60F) die Titelverbindung als gelbes Öl erhalten. Rf = 0,40 (EtOAc-Heptan 1:1); Rt = 3,83.

### d) 4-Benzyl-3-hydroxymethyl-piperazin-1-carbonsäure tert-butyl ester

6,29 g 2-Mercapto-benzoesäure und eine Lösung von 0,57 g 1,4-Bis(diphenylphosphino)butan, 0,78 g Bis(dibenzylidenaceton)palladium in 5 ml Tetrahydrofuran werden zur Lösung von 6,70 g (4-Allyl-1-benzyl-piperazin-2-yl)-methanol in 15 ml Tetrahydrofuran bei Raumtemperatur zugegeben. Nach 30 Minuten wird das Reaktionsgemisch eingedampft - der Rückstand wird mit 120 ml Dioxan, 120 ml 1N NaOH und 7,12 g Di-tert-butyl dicarbonat versetzt und während 16 Stunden bei Raumtemperatur gerührt. Das Reaktionsgemisch wird mit Sole verdünnt und mit Essigester (5x) extrahiert - die vereinigten organischen Phasen werden mit Natriumsulfat getrocknet und eingedampft. Aus dem Rückstand wird mittels Flashchromatographie (SiO₂ 60F) die Titelverbindung als gelber Feststoff erhalten. Rf = 0,22 (EtOAc-Heptan 1:1); Rt = 3,00.

### e) (4-Allyl-1-benzyl-piperazin-2-yl)-methanol

Eine Lösung von 10,70 g 7-Allyl-3-phenyl-tetrahydro-oxazolo[3,4-a]pyrazin-5,8-dion (cis-/trans-Mischung) in 800 ml Tetrahydrofuran wird zur Suspension von 14,64 g Lithiumaluminiumhydrid in 1500 ml Tetrahydrofuran bei Raumtemperatur zugegeben. Das Reaktionsgemisch wird während 19 Stunden am Rückfluss gerührt und anschliessend auf 0 °C abgekühlt. Es werden nacheinander 15 ml Wasser, 15 ml 3 N NaOH und nochmals 15 ml Wasser zugetropft und die Suspension 2 Stunden bei Raumtemperatur ausgerührt. Das Reaktionsgemisch wird über Hyflo klarfiltriert und eingedampft. Der Rückstand wird mit Dichlormethan verdünnt und nacheinander mit 1 N NaOH (2x) und Sole gewaschen, mit Natriumsulfat getrocknet und eingedampft. Aus dem Rückstand wird mittels Flashchromatographie (SiO₂ 60F) die Titelverbindung als gelbes Öl erhalten.
Rf = 0,60 (EtOAc-Ethanol 5:1); Rt = 1,93.

### f) 7-Allyl-3-phenyl-tetrahydro-oxazolof3,4-alpyrazin-5,8-dion (cis-/trans-Mischung)

Eine Lösung von 46 g 3-(2-Chlor-acetyl)-2-phenyl-oxazolidin-4-carbonsäure methyl ester (cis-/trans-Mischung) [Tet. Asym. (2001), 12, 1293-1302], 8,03 g Allylamin und 19,3 ml Triethylamin in 2 l Acetonitril wird während 48 Stunden am Rückfluss gerührt und anschliessend eingedampft. Der Rückstand wird in Essigester aufgenommen und mit 1 N HCl (2x) gewaschen, mit Natriumsulfat getrocknet und eingedampft. Aus dem Rückstand wird mittels Flashchromatographie (SiO₂ 60F) die Titelverbindungen als farblose Öle erhalten. Diastereomer l: Rf = 0,36 (EtOAc-Heptan 1:1); Rt = 3,20.
Diastereomer l: Rf = 0,36 (EtOAc-Heptan 1:1); Rt = 3,08.

### g) 6-lod-4-(3-methoxy-propyl)-4H-benzo[1,4]oxazin-3-on

Eine Mischung von 0,064 g Kupfer(I)iodid, 2,00 g 6-Brom-4-(3-methoxy-propyl)-4H-benzo[1,4]oxazin-3-on und 2,02 g Natriumiodid wird in einer Schlenkapparatur unter Argon vorgelegt, mit 0,088 ml rac-N,N'-Dimethyl-cyclohexan-1,2-diamin und 6 ml Dioxan versetzt und während 19 Stunden bei 110 °C gerührt. Das Reaktionsgemisch wird auf Raumtemperatur abgekühlt, mit wässrigem Ammoniak (30%-ig) verdünnt, ins Wasser gegossen und mit Dichlormethan (3x) extrahiert. Die vereinigten organischen Phasen werden mit Natriumsulfat getrocknet und eingedampft. Aus dem Rückstand wird mittels Flashchromatographie (SiO₂ 60F) die Titelverbindung als gelber Feststoff erhalten.
Rf = 0,55 (EtOAc-Heptan 1:1); Rt = 4,48.

### h) 6-Brom-4-(3-methoxy-propyl)-4H-benzo[1,4]oxazin-3-on

Eine Suspension von 9,87 g 6-Brom-4H-benzo[1,4]oxazin-3-on und 9,52 ml 1-Chlor-3-methoxy-propan, 43,30 g Kaliumfluorid auf Aluminiumoxid, 0,14 g Kaliumiodid und 500 ml Acetonitril wird während 40 Stunden am Rückfluss gerührt. Das Reaktionsgemisch wird abgekühlt, klarfiltriert und das Filtrat zur Trockene eingedampft. Aus dem Rückstand wird mittels Umkristallisation aus Essigester die Titelverbindung als gelb-braune Kristalle erhalten. Rf = 0.43 (EtOAc-Heptan 1:1); Rt = 4,27; Smp. 192-195°C.

### i) 1-(3-(2-Methoxy-benzyloxy)-propoxy)-4-brombenzol

Eine Suspension von 5,1 g 4-Bromphenol, 11,36 g Toluol-4-sulfonsäure 3-(2-methoxybenzyloxy)-propyl ester und 14,13 g Cäsiumcarbonat in 50 ml Acetonitril wird während 6 Stunden bei 85 °C gerührt. Das Reaktionsgemisch wird auf Raumtemperatur abgekühlt, mit gesättigter wässriger Natriumhydrogencarbonat-Lösung verdünnt und mit Dichlormethan (2x) extrahiert. Die vereinigten organischen Phasen werden mit Sole gewaschen, mit Natriumsulfat getrocknet und eingedampft. Aus dem Rückstand wird mittels Flashchromatographie (SiO₂ 60F) die Titelverbindung als weisser Feststoff erhalten.
Rf = 0,58 (EtOAc-Heptan 1:3); Rt = 5,60.

### j) Toluol-4-sulfonsäure 3-(2-methoxy-benzyloxy)-propyl ester

57,04 g p-Toluolsulfonylchlorid werden portionsweise zur Lösung von 50 g 3-(2-Methoxybenzyloxy)-propan-1-ol, 53 ml Triethylamin und 3,25 g 4-Dimethylaminopyridin in 500 ml Dichlormethan bei Raumtemperatur zugegeben. Das Reaktionsgemisch wird während 1 Stunde bei Raumtemperatur gerührt, mit Dichlormethan verdünnt und anschliessend nacheinander mit Wasser und Sole gewaschen, mit Natriumsulfat getrocknet und eingedampft. Aus dem Rückstand wird mittels Flashchromatographie (SiO₂ 60F) die Titelverbindung als weisser Feststoff erhalten. Rf = 0,36 (EtOAc-Heptan 1:3); Rt = 4,93.

### Beispiel 2:

### 6-(1-{4-[3-(2-Methoxy-benzyloxy)-propoxy]-phenyl}-piperazin-2-ylmethoxy)-4-(3-methoxypropyl)-3,4-dihydro-2H-benzo[1,4]oxazin

Analog Methode C wird aus 0,070 g 6-(1-{4-[3-(2-Methoxy-benzyloxy)-propoxy]-phenyl}-piperazin-2-ylmethoxy)-4-(3-methoxy-propyl)-4H-benzo[1,4]oxazin-3-on (Beispiel 1) die Titelverbindung hergestellt.

### Beispiel 3:

### 1-(4-Methoxy-phenyl)-piperazin-2-carbonsäure benzyl cyclopropyl amid

Analog Methode D wird aus 0,0153 g 3-(Benzyl-cyclopropyl-carbamoyl)-4-(4-methoxyphenyl)-piperazin-1-carbonsäure tert-butyl ester die Titelverbindung hergestellt.

Die Ausgangsmaterialien werden wie folgt hergestellt:

### a) 3-(Benzyl-cyclopropyl-carbamoyl)-4-(4-methoxy-phenyl)-piperazin-1-carbonsäure tert-butyl ester

Analog Beispiel 1a werden 0,026 g 4-Bromanisol und 0,0325 g 3-(Benzyl-cyclopropyl-carbamoyl)-piperazin-1-carbonsäure tert-butyl ester umgesetzt. Die Titelverbindung wird als gelbliches Öl erhalten. Rf = 0,40 (EtOAc-Heptan 1:1); Rt = 4,20.

### b) 3-(Benzyl-cyclopropyl-carbamoyl)-piperazin-1-carbonsäure tert-butyl ester

Die Lösung von 0,0685 g 2-(Benzyl-cyclopropyl-carbamoyl)-piperazin-1,4-dicarbonsäure 1-benzyl ester 4-tert-butyl ester in 3 ml Methanol wird in Gegenwart von 0,060 g Pd/C 10 % während 2 Stunden bei Raumtemperatur hydriert. Das Reaktionsgemisch wird klarfiltriert und das Filtrat eingedampft. Aus dem Rückstand wird die rohe Titelverbindung als farbloses Öl erhalten. Rf = 0,05 (EtOAc-Heptan 1:1); Rt = 3,58.

### c) 2-(Benzyl-cyclopropyl-carbamoyl)-piperazin-1,4-dicarbonsäure 1-benzyl ester4-tert-butyl ester

Zu einer Lösung von 0,1 g Piperazin-1,2,4-tricarbonsäure 1-benzyl ester 4-tert-butyl ester in 2 ml Dichlormethan wird bei 0 °C 0,069 ml Chlor-N,N-trimethylpropenylamin zugegeben. Die Lösung wird 3 Stunden bei 0 °C gerührt. Anschliessend wird die Lösung in eine Lösung von 0,062 g Benzyl cyclopropylamin, 0,022 ml Pyridin und 1,6 mg 4-Dimethylamino-pyridin in 2 ml Dichlormethan bei 0 °C kanuliert. Die Reaktionsmischung wird über 10 Stunden langsam auf Raumtemperatur erwärmt. Die Lösung wird mit 3 ml 1 M HCl versetzt und die organische Phase wird abgetrennt. Die wässrige Phase wird mit Dichlormethan extrahiert (2 x 5 ml). Die vereinigten organischen Phasen werden mit Natriumsulfat getrocknet und eingedampft. Aus dem Rückstand wird mittels Flashchromatographie (SiO₂ 60F) die Titelverbindung als farbloses Öl erhalten. Rf = 0,56 (EtOAc-Heptan 1:1); Rt = 5,28.

### d) Piperazin-1,2,4-tricarbonsäure 1-benzyl ester4-tert-butyl ester

Zu einer Mischung von 0,5 g Piperazin-1,3-dicarbonsäure 1-tert-butyl ester, 7 ml Essigester und 7 ml gesättigter wässriger Natriumhydrogencarbonat-Lösung werden 0,37 ml Chlorameisensäure benzyl ester zugegeben. Das Reaktionsgemsich wird 1,5 Stunden bei Raumtemperatur gerührt. Es wird 15 ml Wasser und 15 ml Essigester zugegeben und die Phasen werden getrennt. Die organische Phase wird mit 20 ml Sole gewaschen, mit Natriumsulfat getrocknet und eingedampft. Aus dem Rückstand wird mittels Flashchromatographie (SiO₂ 60F) die Titelverbindung als farbloses Öl erhalten. Rf = 0,25 (EtOAc + 1% AcOH); Rt = 4,12.

### Beispiel 4:

### 1-{4-[2-(2,5-Difluor-phenoxy)-ethoxy]-phenyl}-piperazin-2-carbonsäure (2-chlor-benzyl)-cyclopropyl-amid

Analog Methode D wird aus 0,061 g 3-[(2-Chlor-benzyl)-cyclopropyl-carbamoyl]-4-(4-[2-(2,5-difluor-phenoxy)-ethoxy]-phenyl}-piperazin-1-carbonsäure tert-butyl ester die Titelverbindung hergestellt.

Die Ausgangsmaterialien werden wie folgt hergestellt:

### a) 3-[(2-Chlor-benzyl)-cyclopropyl-carbamoyl]-4-{4-[2-(2,5-difluor-phenoxy)-ethoxy]-phenyl}-piperazin-1-carbonsäure tert-butyl ester

Analog Beispiel 1a werden 0,1 g 3-[(2-Chlor-benzyl)-cyclopropyl-carbamoyl]-piperazin-1-carbonsäure tert-butyl ester und 0.1547 g 2-[2-(4-Brom-phenoxy)-ethoxy]-1,4-difluor-benzol umgesetzt. Die Titelverbindung wird als gelbes Öl erhalten.
Rf = 0,64 (EtOAc-Heptan 1:1); Rt = 5,18.

Bemerkung: statt Tri-tert-butyl phosphin wird Tri-tert-butyl phosphonium tetrafluoroborat als Ligand verwendet.

### b) 3-[(2-Chlor-benzyl)-cyclopropyl-carbamoyl]-piperazin-1-carbonsäure tert-butyl ester

Analog Beispiel 3b werden 1.17 g 2-[(2-Chlor-benzyl)-cyclopropyl-carbamoyl]-piperazin-1,4-dicarbonsäure 1-benzyl ester 4-tert-butyl ester umgesetzt. Die rohe Titelverbindung wird als farbloses Öl erhalten.
Rf = 0,26 (Dichlormethan-Methanol-Ammoniak konz. 25% = 200:10:1); Rt = 3,76.

### c) 2-[(2-Chlor-benzyl)-cyclopropyl-carbamoyl]-piperazin-1,4-dicarbonsäure 1-benzyl ester 4-tert-butyl ester

Analog Beispiel 3c werden 1 g Piperazin-1,2,4-tricarbonsäure 1-benzyl ester 4-tert-butyl ester (Beispiel 3) und 0,8049 g (2-Chlor-benzyl)-cyclopropyl-amin umgesetzt. Die Titelverbindung wird als weisser Schaum erhalten. Rf = 0,29 (EtOAc-Heptan 1:1); Rt = 5,54.

### d) (2-Chlor-benzyl)-cyclopropyl-amin

Zu einer Lösung von 2 g Cyclopropylamin, 4,924 g 2-Chlorbenzaldehyd und 6,8 g Natriumcyanoborhydrid in 25 ml Ethanol wird 2 ml Essigsäure zugegeben. Die Reaktionsmischung wird 16 Stunden bei Raumtemperatur gerührt. Die Reaktionsmischung wird eingedampft. Aus dem Rückstand wird mittels Flashchromatographie (SiO₂ 60F) die Titelverbindung als orangegelbes Öl erhalten.
Rf = 0,60 (Dichlormethan-Methanol-Ammoniak konz. 25% = 200:20:1); Rt = 2,44.

### e) 2-[2-(4-Brom-phenoxy)-ethoxy]-1,4-difluor-benzol

Eine Lösung von 4,78 g Toluol-4-sulfonsäure 2-(2,5-difluoro-phenoxy)-ethyl ester, 3,17 g 4-Bromphenol und 9,11 g Cäsiumcarbonat in 100 ml Acetonitril wird 5 Stunden zum Rückfluss erhitzt. Das Gemisch wird auf Raumtemperatur abgekühlt und auf gesättigte wässrige Natriumhydrogencarbonat-Lösung gegossen. Es wird mit tert-Butyl methyl ether (3 x 50 ml) extrahiert. Die vereinigten organischen Phasen werden mit Natriumsulfat getrocknet und eingedampft. Aus dem Rückstand wird die Titelverbindung als beige Kristalle erhalten. Rf = 0,58 (EtOAc-Heptan 1:1); Rt = 5,33.

### f) Toluol-4-sulfonsäure 2-(2,5-difluor-phenoxy)-ethyl ester

Eine Lösung von 32,6 g Ethylenglykol-bis-(toluol-4-sulfonat), 3,34 g 2,5-Difluor-phenol und 9,15.8 (check) g Cäsiumcarbonat in 120 ml Acetonitril wird 1 Stunden zum Rückfluss erhitzt. Das Gemisch wird auf Raumtemperatur abgekühlt und auf gesättigte wässrige Natriumhydrogencarbonat-Lösung gegossen. Es wird mit tert-Butyl methyl ether (3 x 60 ml) extrahiert. Die vereinigten organischen Phasen werden mit Natriumsulfat getrocknet und eingedampft. Aus dem Rückstand wird mittels Flashchromatographie (SiO₂ 60F) die Titelverbindung als farbloses Oel erhalten. Rf = 0,30 (EtOAc-Heptan 1:2); Rt = 4,79.

### Beispiel 5:

### (rac)-1-{4-[2-(2,5-Difluor-phenoxy)-ethoxy]-phenvl}-piperazin-2-carbonsäure benzylcyclopropyl-amid

Analog Methode D wird aus 0,0893 g (rac)-3-[(Benzyl)-cyclopropyl-carbamoyl]-4-{4-[2-(2,5-difluor-phenoxy)-ethoxy]-phenyl}-piperazin-1-carbonsäure tert-butyl ester die Titelverbindung hergestellt.

Die Ausgangsmaterialien werden wie folgt hergestellt:

### a) (rac)-3-[(Benzyl)-cyclopropyl-carbamoyl]-4-[4-[2-(2,5-difluor-phenoxy)-ethoxyl-phenyl}-piperazin-1-carbonsäure tert-butyl ester

Analog Beispiel 1a werden 0.15 g (rac)-3-[(Benzyl)-cyclopropyl-carbamoyl]-piperazin-1-carbonsäure tert-butyl ester und 0.2541 g 2-[2-(4-Brom-phenoxy)-ethoxy]-1,4-difluor-benzol umgesetzt. Die Titelverbindung wird als gelbes Wachs erhalten.
Rf = 0,50 (EtOAc-Heptan 1:1); Rt = 4,91.
Bemerkung: statt Tri-tert-butyl phosphin wird Tri-tert-butyl phosphonium tetrafluoroborat als Ligand verwendet.

### b) (rac)-3-[(Benzyl)-cyclopropyl-carbamoyl]-piperazin-1-carbonsäure tert-butyl ester

Analog Beispiel 3b werden 1,19 g (rac)-2-[(Benzyl)-cyclopropyl-carbamoyl]-piperazin-1,4-dicarbonsäure 1-benzyl ester 4-tert-butyl ester umgesetzt. Die rohe Titelverbindung wird als farbloses Öl erhalten.
Rf = 0,23 (Dichlormethan-Methanol-Ammoniak konz. 25% = 200:10:1); Rt = 3,66.

### c) (rac)-2-f(Benzyl)-cyclopropyl-carbamoyll-piperazin-1,4-dicarbonsäure 1-benzyl ester 4-tert-butyl ester

Analog Beispiel 3c werden 1 g (rac)-Piperazin-1,2,4-tricarbonsäure 1-benzyl ester 4-tert-butyl ester und 0,6124 g Benzyl cyclopropylamin umgesetzt. Die Titelverbindung wird als farbloses Oel erhalten. Rf = 0,63 (EtOAc-Heptan 1:1); Rt = 5,28.

### Beispiel 6:

### (rac)-1-{4-[3-(2-Methoxy-benzyloxy)-propoxy]-phenyl}-6-[4-(3-methoxy-propyl)-3,4-dihydro-2H-benzo[1,4]oxazin-6-yloxymethyl]-piperazin-2-on

Zu einer Lösung von 0.029 g (rac)-1-{4-[3-(2-Methoxy-benzyloxy)-propoxy]-phenyl}-6-[4-(3-methoxy-propyl)-3,4-d ihydro-2H-benzo[1,4]oxazin-6-yloxymethyl]-4-(toluol-4-sulfonyl)-piperazin-2-on in 2 ml Methanol werden 0.027 g Dinatrium-hydrogenphosphat und 0.086 g 10% Natrium Amalgam zugegeben. Weiteres 10% Natrium Amalgam (0.090 g) werden nach 1.5 Stunden und wieder nach 2.5 Stunden zugegeben. Die Reaktionsmischung wird nach 6 Stunden durch Zugabe von Wasser und Essigester gequencht. Die organische Phase wird mit Natriumsulfat getrocknet und eingedampft. Aus dem Rückstand wird mittels Flashchromatographie (SiO₂ 60F) die Titelverbindung erhalten.

Die Ausgangsmaterialien werden wie folgt hergestellt:

### a) (rac)-1-{4-[3-(2-Methoxy-benzyloxy)-propoxyl-phenyl}-6-[4-(3-methoxy-propyl)-3,4-dihydro-2H-benzo [1,4]oxazin-6-yloxymethyl]-4-(toluol-4-sulfonyl)-piperazin-2-on

Eine Lösung von 0.070 g (rac)-Toluol-4-sulphonsäure 2-[({4-[3-(2-methoxy-benzyioxy)-propoxy]-phenylcarbamoyl}-methyl)-(toluol-4-sulfonyl)-amino]-1-[4-(3-methoxy-propyl)-3,4-dihydro-2H-benzo[1,4]oxazin-6-yloxymethyl]-ethyl ester in 3 ml Tetrahydrofuran wird mit 0.0036 g Natriumhydrid (60% Dispersion in Mineralöl) versetzt. Nach 30 Minuten wird die Reaktionsmischung auf Essigester und 1 M wässrige Kaliumhydrogensulfat-Lösung gegossen. Die organische Phase wird mit Natriumsulfat getrocknet und eingedampft. Aus dem Rückstand wird mittels Flashchromatographie (SiO₂ 60F) die Titelverbindung als braunes Öl erhalten. Rf = 0,37 (2:1 EtOAc-Heptan); Rt = 5,46.

### b) (rac)-Toluol-4-sulphonsäure 2-[({4-[3-(2-methoxy-benzyloxy)-propoxy]-phenylcarbamoyl}-methyl}-(toluol-4-sulfonyl)-amino]-1-[4-(3-methoxy-propyl)-3,4-dihvdro-2H-benzo[1,4]oxazin-6-yloxymethyl]-ethyl ester

Eine Lösung von 0.142 g (rac)-2-{2-Hydroxy-3-[4-(3-methoxy-propyl)-3,4-dihydro-2H-benzo[1,4]oxazin-6-yloxy]-propylamino}-N-(4-[3-(2-methoxy-benzyloxy)-propoxy]-phenyl}-acetamid, 0.184 g p-Toluolsulfonylchlorid, 0.134 ml Triethylamin und 0.001 g Dimethyl-pyridin-4-yl-amin in 5 ml Dichlormethan wird 76 Stunden bei Raumtemperatur gerührt. Die Reaktionsmischung wird eingedampft. Aus dem Rückstand wird mittels Flashchromatographie (SiO₂ 60F) die Titelverbindung als braunes Öl erhalten. Rf = 0,20 (1:1 EtOAc-Heptan); Rt = 5,75.

### c) (rac)-2-{2-Hydroxy-3-[4-(3-methoxy-propyl)-3,4-dihydro-2H-benzo[1,4]oxazin-6-yloxy]-propylamino}-N-{4-[3-(2-methoxy-benzyloxy)-propoxyl-phenyl}-acetamide

Eine Lösung von 0.197 g (rac)-4-(3-Methoxy-propyl)-6-oxiranylmethoxy-3,4-dihydro-2H-benzo[1,4]oxazin, 0.974 g 2-Amino-N-{4-[3-(2-methoxy-benzyloxy)-propoxy]-phenyl}-acetamid und 0.297 ml Triethylamin in 20 ml Methanol wird bei 55°C gerührt. Nach 12 Stunden wird die Reaktionsmischung auf Raumtemperatur abgekühlt und eingedampft. Aus dem Rückstand wird mittels Flashchromatographie (SiO₂ 60F) die Titelverbindung als rot-oranges Öl erhalten. Rf = 0,62 (Dichlormethan-Methanol-25% conc. Ammoniak = 200:20:1); Rt = 4,37.

### d) 2-Amino-N-f4-f3-(2-methoxy-benzyloxy)-propoxyl-phenyl}-acetamid

Eine Lösung von 1.67 g ({4-[3-(2-Methoxy-benzyloxy)-propoxy]-phenylcarbamoyl}-methyl)-carbamidsäure tert-butyl ester in 5 ml Dioxan wird mit 1 ml HCl/Dioxan-Lösung (4 N) versetzt. Nach 2.5 Stunden wird nochmals 1 ml HCl/Dioxan-Lösung (4 N) zugegeben. Es wird über Nacht bei Raumtemperatur gerührt und eingedampft. Aus dem Rückstand wird mittels Flashchromatographie (SiO₂ 60F) die Titelverbindung als gelbes Öl erhalten. Rf = 0,42 (Dichlormethan-Methanol-25% conc. Ammoniak = 200:20:1); Rt = 3,51.

### e) ({4-[3-(2-Methoxy-benzyloxy)-propoxy]-phenylcarbamoyl}-methyl)-carbamidsäure tert-butyl ester

Eine Lösung von 1.10 g 4-[3-(2-Methoxy-benzyloxy)-propoxy]-phenylamin in 50 ml Dichloromethan wird mit 0.684 g tert-Butoxycarbonylamino-essigsäure und 2.71 ml Triethylamin versetzt. Die Reaktionsmischung wird auf 0°C abgekühlt und mit 1.56 ml 2,4,6-Tripropyl-[1,3,5,2,4,6]trioxatriphosphinan-2,4,6-trioxid (50% in Essigester) versetzt. Nach 3 Stunden wird mit tert-Butyl methyl ether verdünnt, mit Wasser und Sole gewaschen, mit Natriumsulfat verdünnt und eingedampft. Titelverbindung wird als rot-braunes Öl erhalten und in der nächsten Stufe ohne weitere Reinigung eingesetzt. Rf = 0,29 (1:1 EtOAc-Heptan); Rt = 4,65.

### f) 4-[3-(2-Methoxy-benzyloxy)-propoxyl-phenylamin

Eine Schlenk-Apparatur wird unter Argon mit einer Mischung aus 2.0 g 1-(3-(2-methoxybenzyloxy)propoxy)-4-brombenzol (Beispiel 1i), 0.064 g Tri-tert-butylphosphin, 0.166 g Bis(dibenzylidenaceton)palladium(0), 6.33 ml Lithium-bis(trimethylsilyl)amid (1M in Hexan) in 15 ml Toluol befüllt. Die Reaktionsmischung wird bei Raumtemperatur gerührt. Nach 16 Stunden wird mit Diethyl ether verdünnt und nacheinander mit 1 N HCl und 1 N NaOH gewaschen, mit Natriumsulfat getrocknet und eingedampft. Aus dem Rückstand wird mittels Flashchromatographie (SiO₂ 60F) die Titelverbindung als dunkelbraunes ÖI erhalten. Rf = 0,30 (1:1 EtOAc-Heptan); Rt = 3,41.

### g) (rac)-4-(3-Methoxy-propyl)-6-oxiranylmethoxy-3,4-dihydro-2H-benzo[1,4]oxazin

Eine Lösung von 4.99 g 4-(3-Methoxy-propyl)-3,4-dihydro-2H-benzo[1,4]oxazin-6-ol in 200 ml N,N-Dimethylformamid wird mit 0.983 g Natriumhydrid (60% Dispersion in Mineralöl) versetzt. Nach 30 Minuten wird portionenweise mit 5.0 g (rac)-Toluol-4-sulfonsäure oxiranylmethyl ester versetzt. Die Reaktionsmischung wird nach 1.5 Stunden mit Wasser verdünnt und mit tert-Butyl methyl ether (3X) extrahiert. Die vereinigten organischen Phasen werden mit Sole gewaschen, mit Natriumsulfat getrocknet und eingedampft. Aus dem Rückstand wird mittels Flashchromatographie (SiO₂ 60F) die Titelverbindung als dunkelbraunes Öl erhalten. Rf = 0,35 (1:1 EtOAc-Heptan); Rt = 3,85.

### h) 4-(3-Methoxy-propyl)-3,4-dihydro-2H-benzo[1,4]oxazin-6-ol

Eine Lösung von 0.527 g 1-(6-Hydroxy-2,3-dihydro-benzo[1,4]oxazin-4-yl)-3-methoxypropan-1-on in 13 ml Tetrahydrofuran wird tropfenweise mit 4.5 ml einer
Boran-Tetrahydrofuran-Lösung (1 M in Tetrahydrofuran) versetzt. Die Reaktionsmischung wird über Nacht gerührt und eingedampft. Der Rückstand mehrfach mit Methanol gestrippt. Aus dem Rückstand wird mittels Flashchromatographie (SiO₂ 60F) die Titelverbindung als hellbraunes Öl erhalten. Rf = 0,33 (1:1 EtOAc-Heptan); Rt = 3,03.

### i) 1-(6-Hydroxy-2,3-dihydro-benzo[1,4]oxazin-4-yl)-3-methoxy-propan-1-on

Eine Suspension von 0.58 g 3,4-Dihydro-2H-benzo[1,4]oxazin-6-ol in 25 ml Dichlormethan wird mit 0.613 ml Pyridin versetzt und auf 0°C abgekühlt. Es wird mit 0.906 ml 3-Methoxypropionylchlorid versetzt und die Reaktionsmischung wird langsam auf Raumtemperatur erwärmt. Nach 2.5 Stunden wird eingedampft und der Rückstand zwischen Essigester und Wasser verteilt. Die organische Phase wird mit 1 N HCl, Wasser und Sole gewaschen, mit Natriumsulfat getrocknet und eingedampft. Die rohe Ester-Amid-Zwischenstufe wird in 30 ml Methanol gelöst und mit 1.2 ml 1 N KOH versetzt. Nach 2 Stunden wird die Reaktionsmischung aufkonzentriert und der Rückstand mit Essigester, Wasser und 1 N HCl verdünnt. Die organische Phase wird mit Wasser und Sole gewaschen, mit Natriumsulfat getrocknet und eingedampft. Aus dem Rückstand wird mittels Flashchromatographie (SiO₂ 60F) die Titelverbindung als hellbraunes Öl erhalten. Rf = 0,54 (EtOAc); Rt = 2,62.

Nach dem im Beispiel 6 beschriebenen Verfahren wird in analoger Weise die nachfolgende Verbindung hergestellt:

### Beispiel 7:

### 1-{4-[1-(3-Fluor-phenyl)-pyrrolidin-3(S)-yloxy]-phenyl}-6(R,S)-[4-(3-methoxy-propyl)-3.4-dihydro-2H-benzo[1,4]oxazin-6-yloxymethyl]-piperazin-2-on

Das Ausgangsmaterial wird wie folgt hergestellt:

### a) (R)-1-(3-Fluor-phenyl)-pyrrolidin-3-ol

Analog zu Beispiel 1a, werden 2.96 g (R)-Pyrrolidin-3-ol und 3.0 ml 1-Brom-3-fluor-benzol umgesetzt. Die Titelverbindung wird als dunkelbraunes Öl erhalten.
Rf = 0,22 (2:3 EtOAc-Heptan); Rt = 3,55.
Bemerkung: statt Tri-tert-butylphosphin, wird (rac)-2,2'-Bis(diphenylphosphin)-1,1'-binaphthyl als Ligand verwendet.

| **Nr.** | **Struktur** | **Aspekt** | **R**_{**f**}**(System)** | **Rt (Method)** |
|---|---|---|---|---|
| 1 | 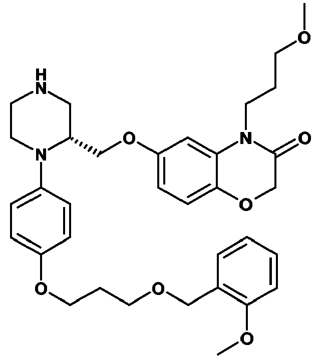 | gelbes Öl | 0.36 (A) | 4.12 (I) |
| 2 | 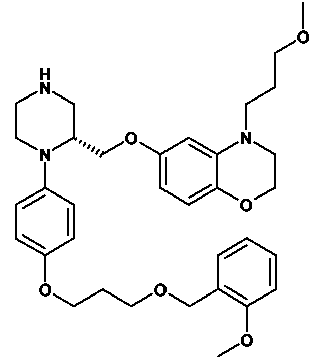 | gelb-braunes Öl | 0.25 (A) | 4.45 (I) |
| 3 | 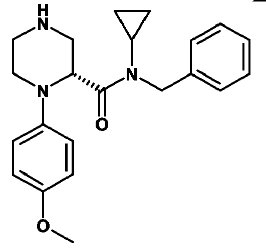 | gelbliches Öl | 0.38 (A) | 3.52 (I) |
| 4 | 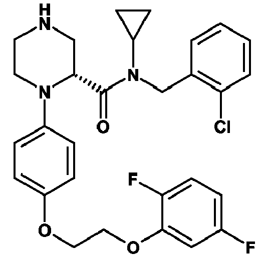 | weisser Feststoff | 0.47 (A) | 4.60 (I) |
| 5 | 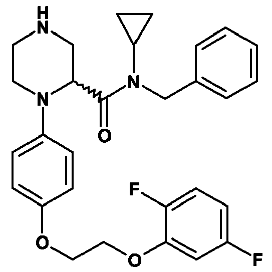 | weisser Feststoff | 0.38 (A) | 4.38 (I) |
| 6 | 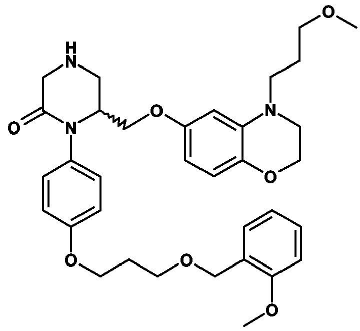 | gelbes Öl | 0.32 (A) | 4.29 (I) |
| 7 | 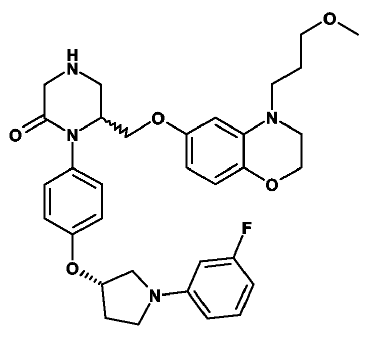 | farbloses Öl | 0.22 (A) | 4.54 (I) |

Dünnschichtchromatographie Fliessmittelsysteme :
A Dichlormethan-Methanol-Ammoniak konz. 25% = 200:20:1
B Dichlormethan-Methanol-Ammoniak konz. 25% = 200:20:0.5
C Dichlormethan-Methanol-Ammoniak konz. 25% = 200:10:1
D Dichlormethan-Methanol-Ammoniak konz. 25% = 90:10:1
E Dichlormethan-Methanol-Wasser-Essigsäure konz. = 750:270:50:5

## Patentansprüche

1. Verwendung einer Verbindung der allgemeinen Formel (I) oder (II) 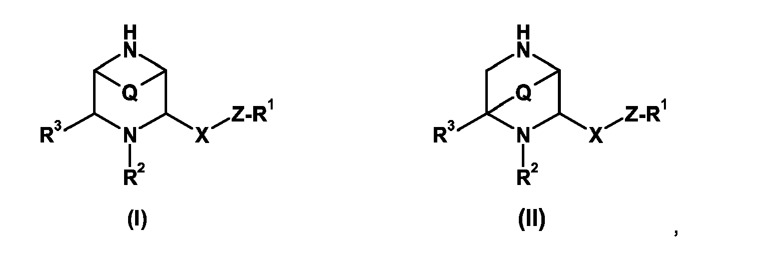 worin
R¹ Aryl oder Heterocyclyl;
R² Phenyl, Naphthyl, Acenaphthyl, Cyclohexyl, Pyridyl, Pyrimidinyl, Pyrazinyl, Oxo-pyridinyl, Diazinyl, Triazolyl, Thienyl, Oxazolyl, Oxadiazolyl, Thiazolyl, Pyrrolyl, Furyl, Tetrazolyl oder Imidazolyl, welche Reste durch 1-3 Halogen, Hydroxy, Cyano, Trifluormethyl, C₁₋₆-Alkyl, Halo-C₁₋₆-alkyl, Hydroxy-C₁₋₆-alkyl, C₁₋₆-Alkoxy-C₁₋₆-alkyl, Cyano-C₁₋₆-alkyl, Carboxy-C₁₋₆₋alkyl, C₁₋₆-Alkanoyloxy-C₁₋₆-alkyl, C₁₋₆-Alkoxycarbonyloxy-C₁₋₆-alkyl, C₁₋₆-Alkoxycarbonyl, oder C₁₋₆-Alkoxygruppen, oder eine C₁₋₆-Alkylendioxygruppe, und/oder durch einen Rest L1-T1-L2-T2-L3-T3-L4-T4-L5-U substituiert sein können, darstellen;
L1, L2, L3, L4 und L5 unabhängig voneinander eine Bindung, C₁₋₈-Alkylen, C₂₋₈-Alkenylen oder C₂₋₈-Alkinylen darstellen, oder abwesend sind;
T1, T2, T3 und T4 unabhängig voneinander
(a) eine Bindung darstellen, oder abwesend sind, oder eine der Gruppen
(b) -CH(OH)-,
(c) -CH(OR⁶)-,
(d) -CH(NR⁵R⁶)-,
(e) -CO-,
(f) -CR⁷R⁸-,
(g) -O- oder -NR⁶-,
(h) -S(O)₀₋₂-,
(I) -SO₂NR⁶-,
(j) -NR⁶SO₂-,
(k) -CONR⁶-,
(I) -NR⁶CO-,
(m) -O-CO-,
(n) -CO-O-,
(o) -O-CO-O-,
(p) -O-CO-NR⁶-,
(q) -N(R⁶)-CO-N(R⁶)-,
(r) -N(R⁶)-CO-O-,
(s) Pyrrolidinylen, Piperidinylen oder Piperazinylen, und
(t) -C(R¹¹)(R¹²)- darstellen,
wobei die von (b)-(t) ausgehenden Bindungen zu einem gesättigten oder aromatischen C-Atom der benachbarten Gruppe führen, falls die Bindung von einem Heteroatom ausgeht, und wobei nicht mehr als zwei Gruppen (b)-(f), drei Gruppen (g)-(h) und eine Gruppe (i)-(t) anwesend sind;
R³ Wasserstoff, C₁₋₆-Alkyl, C₂₋₆-Alkenyl, C₁₋₆-Alkoxy, Hydroxy-C₁₋₆-alkyl, C₁₋₆-Alkoxy-C₁₋₆-alkyl, Benzyl, Oxo, oder eine Gruppe
R⁴-Z1-X1- darstellen, wobei R⁴
(a) H-,
(b) C₁₋₆-Alkyl-,
(c) C₂₋₆-Alkenyl-,
(d) Hydroxy-C₁₋₆-alkyl-,
(e) Polyhydroxy-C₁₋₆-alkyl-,
(f) C₁₋₆-Alkyl-O-C₁₋₆-alkyl-,
(g) Aryl-,
(h) Heterocyclyl-,
(i) Arylalkyl-,
(j) Heterocyclylalkyl-,
(k) Aryloxyalkyl-,
(l) Heterocyclyloxyalkyl-,
(m) (R⁵,R⁶)N-(CH₂)₁₋₃-,
(n) (R⁵,R⁶)N-,
(o) C₁₋₆-Alkyl-S(O)₀₋₂-,
(p) Aryl-S(O)₀-₂-,
(q) Heterocyclyl-S(O)₀₋₂-,
(r) HO-SO₃- bzw. deren Salze,
(s) H₂N-C(NH)-NH-, und
(t) NC- darstellt, und die von (n)-(t) ausgehenden Bindungen zu einem C-Atom der benachbarten Gruppe führen und dieses C-Atom gesättigt ist, falls die Bindung von einem Heteroatom ausgeht;
Z1
(a) eine Bindung darstellt, abwesend ist, oder eine der Gruppen
(b) C₁₋₆-Alkylen-,
(c) C₂₋₆-Alkenylen-,
(d) -O-, -N(R¹¹)-, -S(O)₀₋₂-,
(e) -CO-,
(f) -O-CO-,
(g) -O-CO-O-,
(h) -O-CO-N(R¹¹)-,
(i) -N(R¹¹)-CO-O-,
(j) -CO-N(R¹¹)-,
(k) -N(R¹¹)-CO-,
(l) -N(R¹¹)-CO-N(R¹¹)-, und
(m) -CH(OR⁹)-
darstellt, und die von (d) und (f)-(m) ausgehenden Bindungen zu einem C-Atom der benachbarten Gruppe führen und dieses C-Atom gesättigt ist, falls die Bindung von einem Heteroatom ausgeht;
X1 eine Bindung darstellt, abwesend ist, oder -(CH₂)₁₋₃- darstellt;
R⁵ und R⁶ Wasserstoff, C₁₋₆-Alkyl, C₂₋₆-Alkenyl, Aryl-C₁₋₆-alkyl oder Acyl bedeuten, oder gemeinsam mit dem N-Atom, an das sie gebunden sind, einen 5- oder 6-gliedrigen heterocyclischen Ring bedeuten, der ein zusätzliches N-, O- oder S-Atom oder eine -SO-oder -SO₂- Gruppe enthalten kann, wobei das zusätzliche N-Atom gegebenenfalls durch C₁₋₆-Alkyl-Reste substituiert sein kann;
R⁷ und R⁸ gemeinsam mit dem C-Atom, an das sie gebunden sind, einen 3-7-gliedrigen Ring darstellen, der ein oder zwei -O- oder -S- Atome oder -SO- oder -SO₂- Gruppen enthalten kann;
R⁹ Wasserstoff, C₁₋₆-Alkyl, C₃₋₈-Cycloalkyl, C₁₋₆-Alkoxy-C₁₋₆-alkyl, Acyl oder Arylalkyl;
R¹⁰ Carboxyalkyl, Alkoxycarbonylalkyl, Alkyl oder Wasserstoff;
R¹¹ Wasserstoff oder C₁₋₆-Alkyl ist;
R¹² Wasserstoff oder C₁₋₆-Alkyl ist;
U Wasserstoff, C₁₋₆-alkyl, Cycloalkyl, Cyano, gegebenenfalls substituiertes Cycloalkyl, Aryl, oder Heterocyclyl darstellt;
Q Ethylen darstellt oder abwesend ist (Formel I) oder Ethylen oder Methylen darstellt (Formel II);
X eine Bindung oder eine Gruppe >CH-R¹¹, >CR⁹R¹¹, >CHOR⁹, >CO oder >C=NOR¹⁰ darstellt;
Z abwesend ist oder C₁₋₆-Alkylen, C₂₋₆-Alkenylen, Hydroxy-C₁₋₆-alkyliden, -CH-R¹¹-CO-NR⁹-, - O-, -S-, -NR⁹, -O-Alk-, -S-Alk-, -NR⁹-Alk-, -Alk-O-, -Alk-S- oder -Alk-NR⁹- ist, wobei Alk C₁₋₆₋Alkylen bezeichnet; und wobei
(a) falls Z -O- oder -S- darstellt, X -CR⁹R¹¹- ist und entweder R² einen Substituenten L1-T1-L2-T2-L3-T3-L4-T4-L5-U enthält oder R³ ein wie oben definierter, von Wasserstoff verschiedener Substituent ist;
(b) falls Z -O-Alk-, -S-Alk- oder -NR⁹-Alk- darstellt, X -CR⁹R¹¹- ist; und
(c) falls X eine Bindung darstellt, Z C₁₋₆-Alkenylen, -Alk-O-, Alk-S- oder -Alk-NR⁹-darstellt, und ihr Salz, Prodrug oder Verbindung, bei welcher ein oder mehrere Atome durch ihre stabilen, nicht radioaktiven Isotope ersetzt sind, insbesondere pharmazeutisch anwendbares Salz;
zur Herstellung eines Humanheilmittels zur Beta-Sekretase-, Cathepsin D-, Plasmepsin II-und/oder HIV-Protease-Hemmung.

2. Verwendung gemäss Anspruch 1, **dadurch gekennzeichnet, dass** die Verbindung der Formel (IA) oder (IIA) 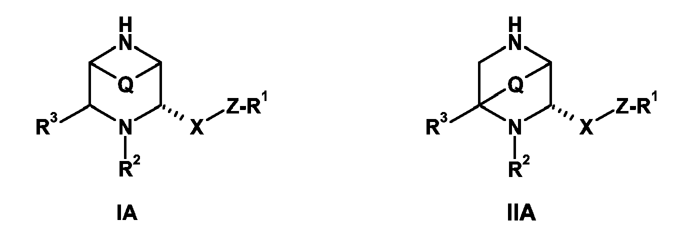 worin R¹, R², R³, Q, X und Z die für die Verbindungen der Formeln (I) bzw. (II) gemäss Anspruch 1 angegebene Bedeutung haben.

3. Verfahren zur Beta-Sekretase-, Cathepsin D-, Plasmepsin 11- und/oder HIV-Protease-Hemmung, wobei eine therapeutisch wirksame Menge einer Verbindung der allgemeinen Formel (I), (II), (IA) oder (IIA), gemäss den Ansprüchen 1 und 2, verwendet wird.

4. Pharmazeutisches Präparat zur Beta-Sekretase-, Cathepsin D-, Plasmepsin 11- und/oder HIV-Protease-Hemmung enthaltend eine Verbindung der allgemeinen Formel (I), (II), (IA) oder (IIA), gemäss den Ansprüchen 1 und 2, sowie übliche Hilfsstoffe.

5. Verwendung einer Verbindung der allgemeinen Formel (I), (II), (IA) oder (IIA), gemäss den Ansprüchen 1 und 2, zur Herstellung eines Humanheilmittels zur Prevention, zur Progressionsverzögerung oder zur Behandlung von Alzheimer, Malaria oder HIV.

6. Verfahren zur Prevention, zur Progressionsverzögerung oder zur Behandlung von Alzheimer, Malaria oder HIV, wobei eine therapeutisch wirksame Menge einer Verbindung der allgemeinen Formel (I), (II), (IA) oder (IIA), gemäss den Ansprüchen 1 und 2, verwendet wird.

7. Pharmazeutisches Präparat zur Progressionsverzögerung oder zur Behandlung von Alzheimer, Malaria oder HIV enthaltend eine Verbindung der allgemeinen Formel (I), (II), (IA) oder (IIA), gemäss den Ansprüchen 1 und 2, sowie übliche Hilfsstoffe.
